# EUROPEAN PATENT SPECIFICATION

(11) **EP 2 406 373 B1**
(45) Date of publication and mention of the grant of the patent: **28.05.2014**
(21) Application number: 10708843.7
(22) Date of filing: 24.02.2010
(51) Int. Cl.: C12N 9/28, C12R 1/11

(54) **BACILLUS MEGATERIUM STRAIN DSM90-RELATED ALPHA-AMYLASES, AND METHODS OF USE, THEREOF**
ALPHA-AMYLASE AUS BACILLUS MEGATERIUM DSM90, VERWANDTE AMYLASEN UND DEREN VERWENDUNG
ALPHA AMYLASE DE BACILLUS MEGATERIUM DSM90, AMYLASES APPARENTÉES ET LEURS USAGES

(30) Priority: 10.03.2009 US 158950 P
(43) Date of publication of application: 18.01.2012
(73) Proprietor: Danisco US Inc., Palo Alto, CA 94304 (US)
(72) Inventor: JONES, Brian E., Palo Alto, California 94304 (US); KOLKMAN, Marc, Palo Alto, California 94304 (US)
(74) Representative: Forrest, Graham Robert
(86) International application number: PCT/US2010/025174
(87) International publication number: WO 2010/104675

(56) References cited:
- WO-A1-99/19467
- WO-A2-02/068589
- WO-A2-2004/091544
- JP-T- 2007 525 158
- KR-A- 20040 006 812
- DATABASE UniProt [Online] 10 February 2009 (2009-02-10), "SubName: Full=Alpha-amylase; EC=<A HREF="http://srs.ebi.ac.uk/srsbin/cgi-bin/ wgetz?[enzyme-ECNumber:3.2.1.1]+-e">3.2.1. 1</A>;" XP002580500 retrieved from EBI accession no. UNIPROT:B7IQL5 Database accession no. B7IQL5
- DATABASE UniProt [Online] 25 November 2008 (2008-11-25), "SubName: Full=Alpha-amylase; EC=<A HREF="http://srs.ebi.ac.uk/srsbin/cgi-bin/ wgetz?[enzyme-ECNumber:3.2.1.1]+-e">3.2.1. 1</A>;" XP002580501 retrieved from EBI accession no. UNIPROT:B5UL87 Database accession no. B5UL87
- DATABASE UniProt [Online] 23 September 2008 (2008-09-23), "SubName: Full=Alpha-amylase; EC=<A HREF="http://srs.ebi.ac.uk/srsbin/cgi-bin/ wgetz?[enzyme-ECNumber:3.2.1.1]+-e">3.2.1. 1</A>;" XP002592634 retrieved from EBI accession no. UNIPROT:B3Z5Z5 Database accession no. B3Z5Z5

## Description

### TECHNICAL FIELD

Disclosed are compositions and methods relating to α-amylase enzymes obtained from *Bacillus megaterium* strain DSM90, and to structurally related amylases.

### BACKGROUND

Starch consists of a mixture of amylose (15-30% w/w) and amylopectin (70-85% w/w). Amylose consists of linear chains of α-1,4-linked glucose units having a molecular weight (MW) from about 60,000 to about 800,000. Amylopectin is a branched polymer containing α-1,6 branch points every 24-30 glucose units; its MW may be as high as 100 million.

Sugars from starch, in the form of concentrated dextrose syrups, are currently produced by an enzyme catalyzed process involving: (1) liquefaction (or viscosity reduction) of solid starch with an α-amylase into dextrins having an average degree of polymerization of about 7-10, and (2) saccharification of the resulting liquefied starch (*i.e.* starch hydrolysate) with amyloglucosidase (also called glucoamylase or GA). The resulting syrup has a high glucose content. Much of the glucose syrup that is commercially produced is subsequently enzymatically isomerized to a dextrose/fructose mixture known as isosyrup.

α-amylases (EC 3.2.1.1) hydrolyze starch, glycogen, and related polysaccharides by cleaving internal α-1,4-glucosidic bonds at random. α-amylases, particularly from *Bacilli,* have been used for a variety of different purposes, including starch liquefaction, textile desizing, starch modification in the paper and pulp industry, and for brewing. These enzymes can also be used to remove starchy stains during dishwashing and laundry washing. The need exists for α-amylases with superior cleaning properties.

### SUMMARY

The present compositions and methods as defined in claims 1-16 relate to an α-amylase from *Bacillus megaterium* strain DSM90 and related α-amylases, which represent a unique family of amylases useful for industrial applications. These amylases are referred to collectively as AmyDSM90-related polypeptides or AmyDSM90-related amylases.

In one aspect, an isolated α-amylase polypeptide having at least 80% amino acid sequence identity to AmyDSM90 (SEQ ID NO: 1) is provided, wherein the polypeptide has:
a) an aspartic acid at a position corresponding to position 21 of AmyDSM90 (SEQ ID NO: 1);
b) an asparagine at a position corresponding to position 97 of AmyDSM90 (SEQ ID NO: 1); and
c) an isoleucine at a position corresponding to position 128 of AmyDSM90 (SEQ ID NO: 1).

In some embodiments, the α-amylase polypeptide is expressed by a heterologous cell as a secreted polypeptide.

In some embodiments, the polypeptide has at least 90% identity to the amino acid sequence of SEQ ID NO: 1. In some embodiments, the polypeptide has at least 95% identity to the amino acid sequence of SEQ ID NO: 1. In some embodiments, the polypeptide has the amino acid sequence of SEQ ID NO: 1. In some embodiments, the polypeptide has the amino acid sequence of SEQ ID NO: 28, SEQ ID NO: 29, or SEQ ID NO: 30.

In a further aspect, a composition comprising any of the aforementioned polypeptides is provided.

In some embodiments, the composition is a cleaning composition. In some embodiments, the composition is effective for removing starchy stains from laundry. In some embodiments, the composition is effective for removing starchy stains from dishes. In some embodiments, the composition is effective for removing starchy stains from a textile.

In another aspect, a method for removing a starchy stain from a surface is provided, comprising incubating the surface in the presence of a aqueous composition comprising an effective amount of an α-amylase as described above, and allowing the α-amylase to hydrolyse starch components present in the starchy stain to produce smaller starch-derived molecules that dissolve in the aqueous composition, thereby removing the starchy stain from the surface.

In some embodiments, the surface is a textile surface. In some embodiments, the surface is on dishes. In some embodiments, the surface is a laundry surface.

In another aspect, polynucleotides encoding the aforementioned polypeptides are provided, along with expression vectors that include such polynucleotides. In yet a further aspect, host cells that include these expression vectors are provided.

In another aspect, a method for expressing an α-amylase is provided, comprising: providing a host cell comprising an expression vector as described above, wherein the polynucleotide encoding the α-amylase is fused in-frame to a signal sequence; expressing the α-amylase as a secreted polypeptide into host cell media; and recovering the secreted α-amylase from the host cell growth media; thereby isolating the α-amylase as a secreted polypeptide.

In some embodiments, the signal sequence is the native signal sequence. In some embodiments, the signal sequence is from Bacillus AmyE or AprE or Streptomyces CelA.

These and other aspects and embodiments of the compositions and methods as defined in claims 1-16 will be apparent from the present description and drawings.

### BRIEF DESCRIPTION OF THE DRAWINGS

FIG. 1. Alignment of protein sequences from *B. megaterium* DSM90 (AmyDSM90, SEQ ID NO: 1) and *B. megaterium* AAK00598 (SEQ ID NO: 3).
FIG. 2. Alignment of orthologous amylase sequences from *B. megaterium* DSM90 (AmyDSM90, SEQ ID NO: 1), *B. anthracis* AAT32659 (SEQ ID NO: 4), *B. thuringiensis* AAT60457 (SEQ ID NO: 5), *B. cereus* AAP10417 (SEQ ID NO: 6), and *B.megaterium* AAK00598 (SEQ ID NO: 3).
FIG. 3. Schematic diagram of plasmid vector pHPLT containing the thermostable amylase LAT promoter (pLAT) and the LAT signal peptide (pre LAT) of *Bacillus licheniformis,* followed by *Pst*I and *Hpa*I restriction sites for cloning.
FIG. 4. Schematic diagram of plasmid pME602.13 (also called pHPLT-B.meg DSM90 Amy) containing the amylase gene amplified from strain DSM90.
FIG. 5. Gene construct for AmyDSM90 expression.
FIG. 6. Nucleotide (SEQ ID NO: 9) and corresponding amino acid sequence (SEQ ID NO: 10) of AmyDSM90 expressed in *B. licheniformis* as a fusion protein with the signal peptide of *B. licheniformis* α-amylase (LAT).
FIG. 7. Schematic diagram of pICatH-B.meg DSM90 amy plasmid containing the LAT-DSM90 amylase gene.
FIG. 8. Graph showing the cleaning performance of AmyDSM90 using CS-28 rice starch stained fabric swatches in 25 mM BTP pH 8 buffer at 20°C for 1 hour.
FIG. 9. Graph showing the cleaning performance of AmyDSM90 using CS-28 rice starch stained fabric swatches in 25 mM BTP pH 8 buffer at 40°C for 1 hour.
FIG. 10. Graph showing the cleaning perfonnance of AmyDSM90 using CS-28 rice starch stained fabric swatches in 25 mM CAPS pH 10.3 buffer at 20°C for 1 hour.
FIG. 11. Graph showing the cleaning performance of AmyDSM90 using CS-28 rice starch stained fabric swatches in 25 mM CAPS pH 10.3 buffer at 40°C for 1 hour.
FIG. 12. Graph showing the cleaning of CS-28 rice starch-stained fabric swatches by AmyDSM90-related polypeptides in 25 mM HEPES buffer, pH 8.
FIG. 13. Graph showing the cleaning of CS-28 rice starch-stained fabric swatches by AmyDSM90-related polypeptides in 25 mM CAPS buffer, pH 10.
FIG. 14. Graph showing the laundry-wash performance of *B. megaterium* amylases using CS-28 rice starch-stained fabric swatches in AATCC powder detergent, pH 10.5.
FIG. 15. Graph showing the laundry-wash performance of *B. megaterium* amylases using CS-28 rice starch-stained fabric swatches in AATCC liquid detergent, pH 7.
FIG. 16. Graph showing the cleaning performance of AmyDSM90 as a function of dosage in Terg-o-tometer experiments using CS-28 rice starch stained fabric swatches in AATCC powder detergent, pH 10 at 40°C.
FIG. 17. Graph showing the washing performance of Amy DSM90 in a commercial gel detergent compared to commercial amylases.
FIG. 18. Graph showing the washing performance of AmyDSM90 in a standard detergent compared to commercial amylase.
FIG. 19. Graph showing the washing performance of AmyDSM90-related amylases in a commercial gel detergent.
FIG. 20. Graph showing the washing performance of AmyDSM90-related amylases in a standard detergent containing bleach.
FIG. 21. Graph showing the washing performance of AmyDSM90-related amylases in a standard detergent without bleach.

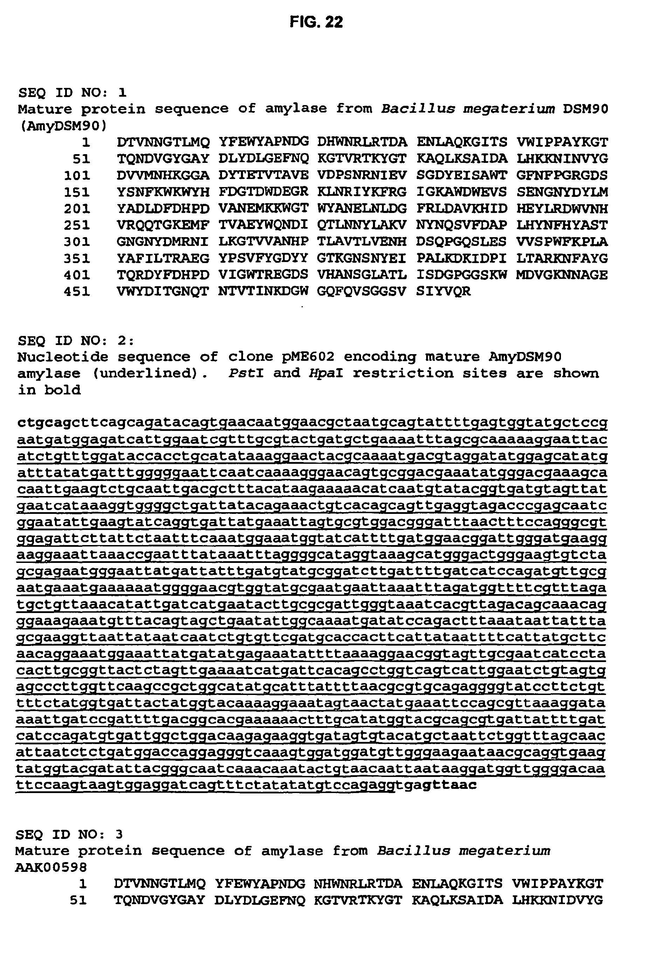

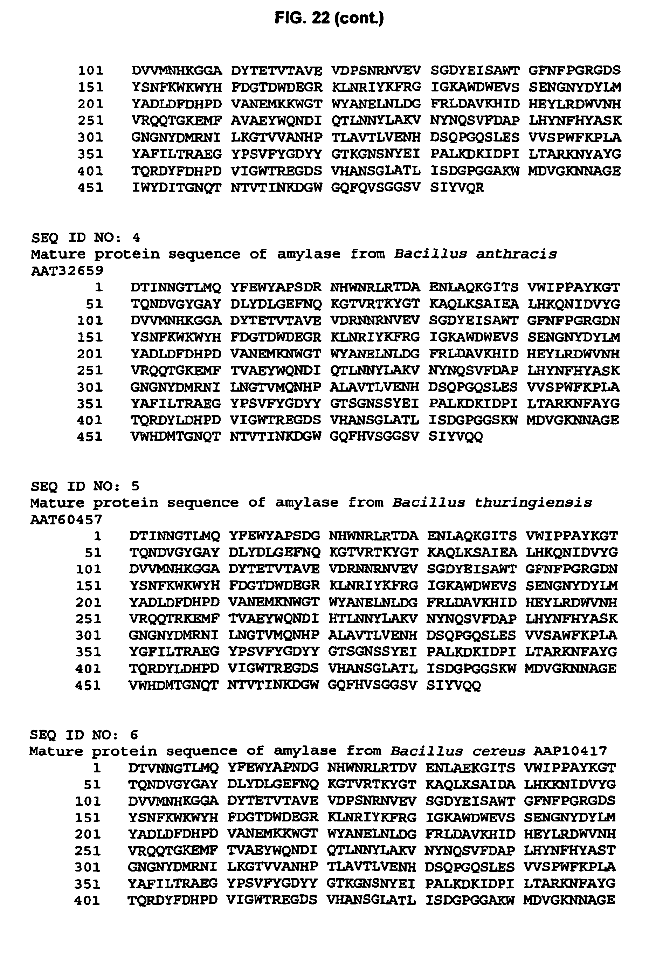

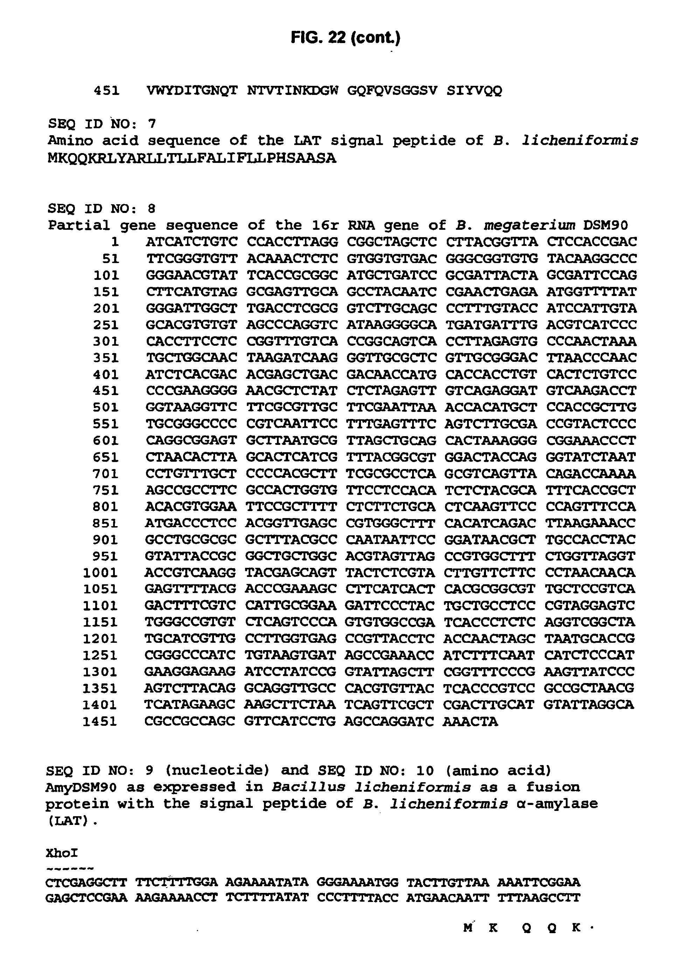

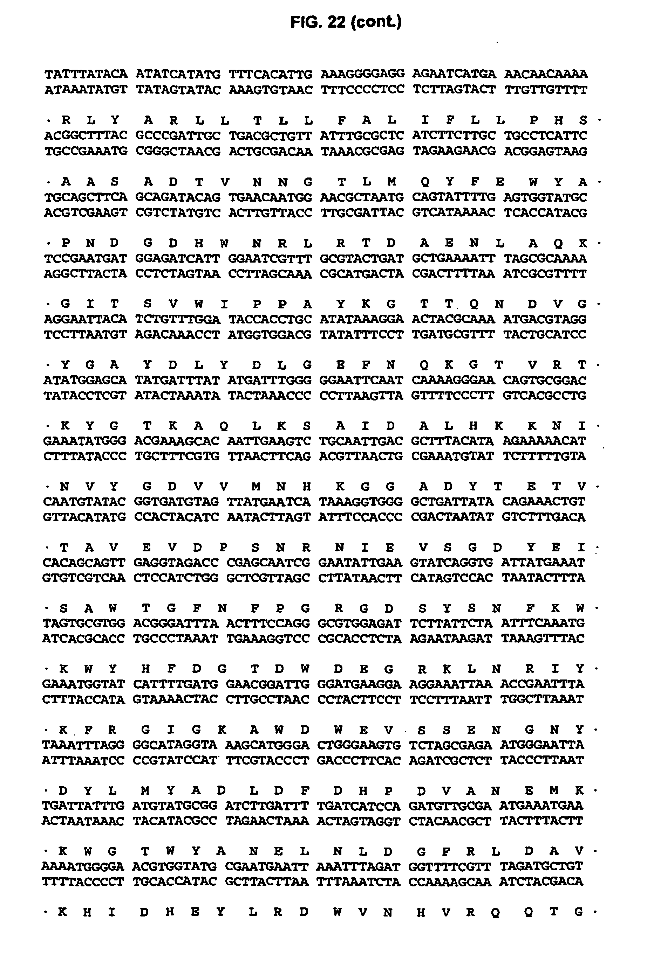

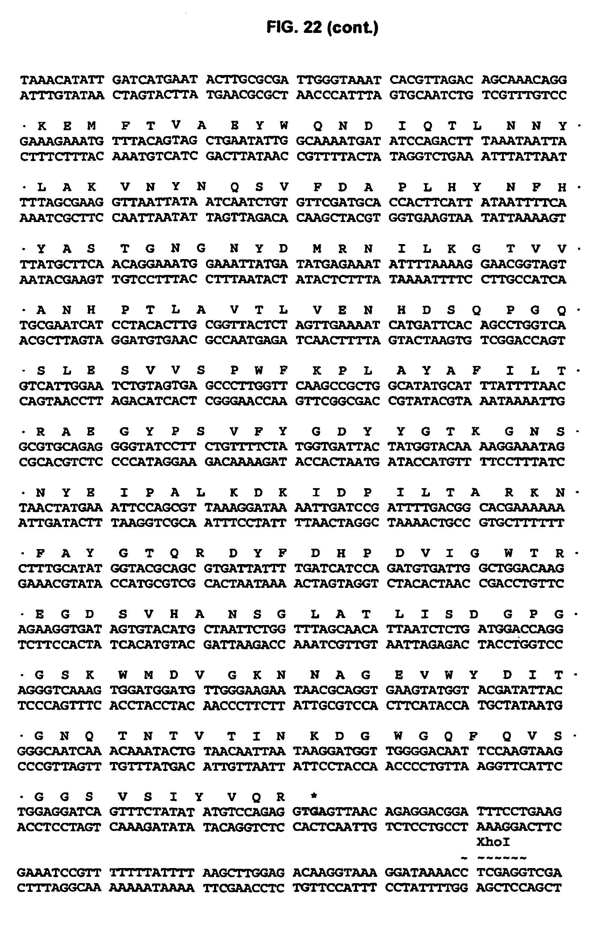

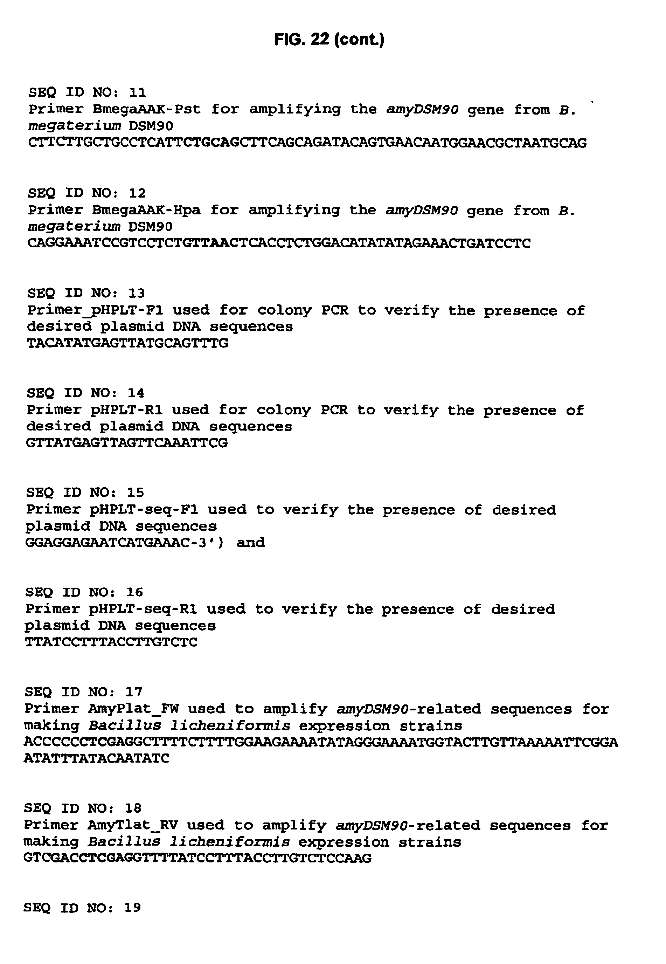

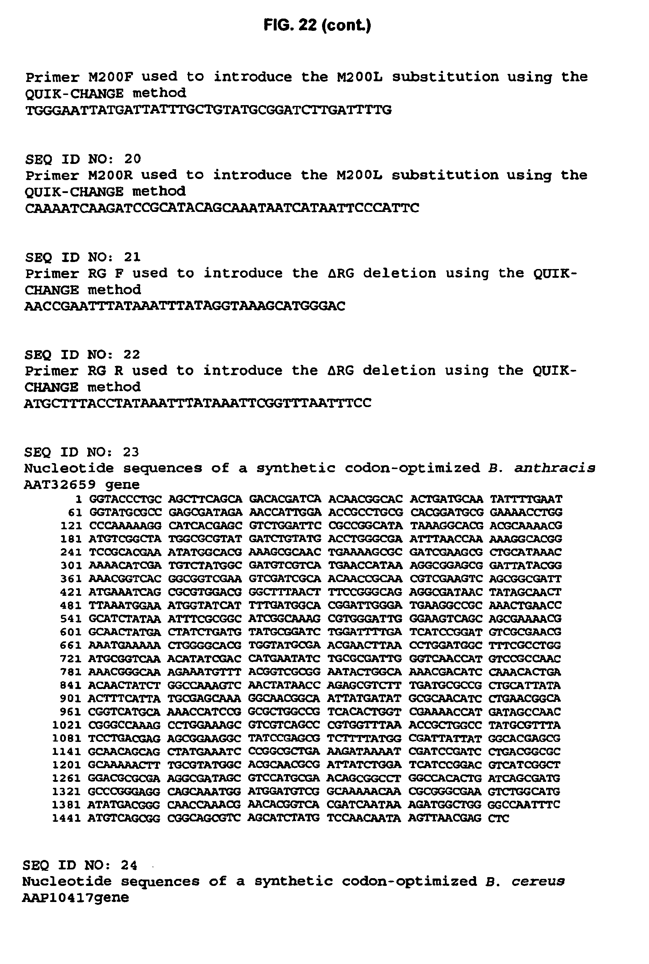

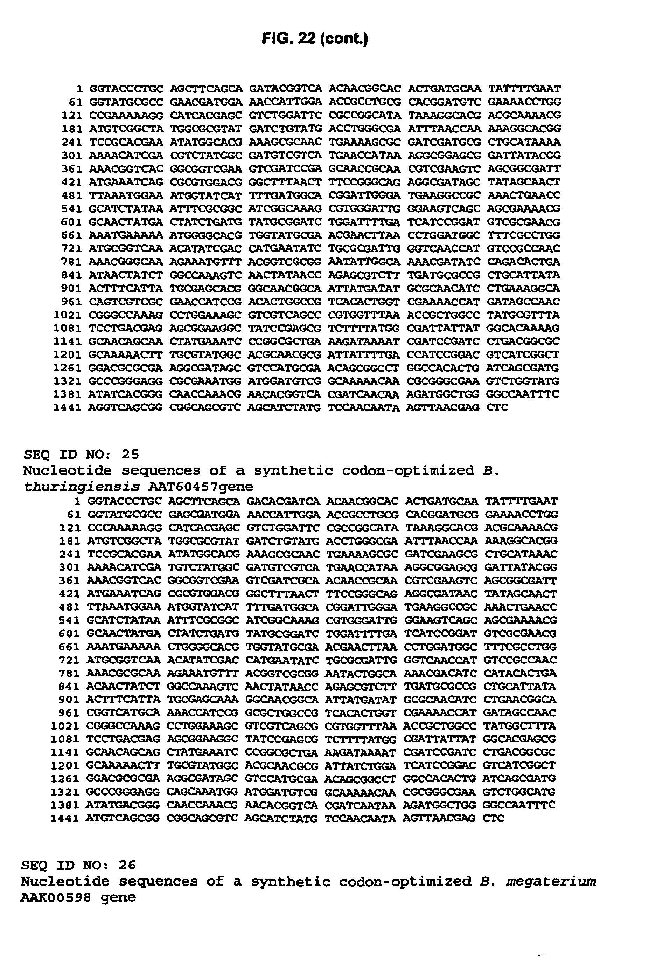

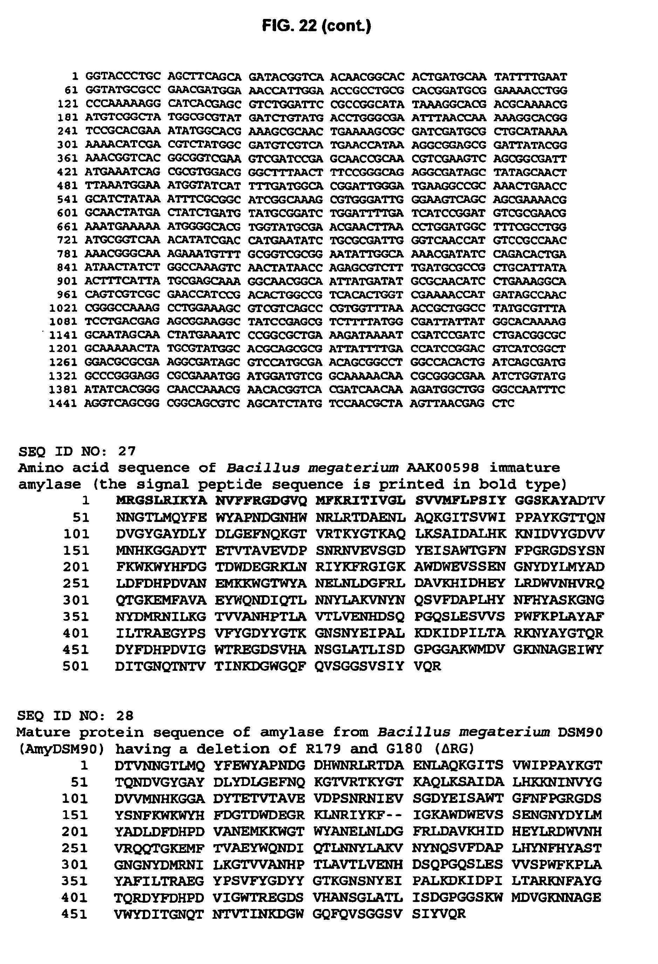

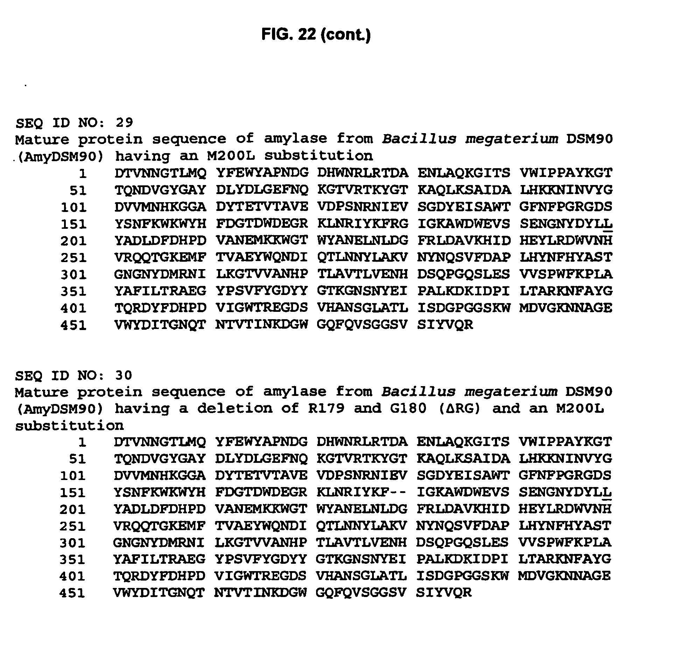

FIG. 22. Polypeptide and nucleotide sequences referred to in the description.

### BRIEF DESCRIPTION OF THE SEQUENCES

SEQ ID NO: I is the amino acid sequence of the mature *B. megaterium* AmyDSM90 amylase.
SEQ ID NO; 2 is the nucleotide sequence of clone pME602.
SEQ ID NO:: 3 is the amino acid sequence of the mature *B. megaterium* AAK00598 amylase.
SEQ ID NO: 4 is the amino acid sequence of the mature *B. anthracis* AAT32659 amylase.
SEQ ID NO: 5 is the amino acid sequence of the mature *B. thuringiensis* AAT60457 amylase.
SEQ ID NO: 6 is the amino acid sequence of the mature *B. cereus* AAP10417 amylase.
SEQ ID NO: 7 is the amino acid sequence of the LAT signal peptide of *B. licheniformis.*
SEQ ID NO: 8 is a partial nucleotide sequence of the *B. megaterium* DSM90 16S rRNA.
SEQ ID NO: 9 is the nucleotide sequence of the polynucleotide encoding AmyDSM90 expressed in *B. licheniformis* as a fusion protein with the signal peptide of *B. licheniformis α-*amylase (LAT).
SEQ ID NO: 10 is the amino acid sequence of the AmyDSM90 polypeptides expressed in *B. lichenformis* as a fusion protein with the signal peptide of *B. licheniformis* α-amylase (LAT).
SEQ ID NOs: 11 and 12 are the nucleotide sequences of PCR primers used to amplify the *amyDSM90* gene from *B. megaterium* DSM90.
SEQ ID NOs: 13 and 14 are the nucleotide sequences of PCR primers used for colony PCR to verify the presence of desired plasmid DNA sequences.
SEQ ID NOs: 15 and 16 are the nucleotide sequences of sequencing primers used to verify the presence of desired plasmid DNA sequences.
SEQ ID NOs: 17 and 18 are the nucleotide sequences of PCR primers used to amplify *amyDSM90*-related sequences for making *B. licheniformis* expression strains.
SEQ ID NOs: 19 and 20 are the nucleotide sequences of primers used to introduce the M200L substitution using the QUIK-CHANGE methods.
SEQ ID NOs: 21 and 22 are the nucleotide sequences of primers used to introduce the ΔRG deletion using the QUIK-CHANGE method.
SEQ ID NO: 23 is the nucleotide sequence of a synthetic gene encoding an amylase from *B. anthracis* AAT32659.
SEQ ID NO: 24 is the nucleotide sequence of a synthetic gene encoding an amylase from *B. cereus* AAP10417.
SEQ ID NO: 25 is the nucleotide sequence of a synthetic gene encoding an amylase from *B. thuringiensis* AAT60457.
SEQ ID NO: 26 is the nucleotide sequence of a synthetic gene encoding an amylase from *B. megaterium* AAK00598.
SEQ ID NO: 27 is the amino acid sequence of the immature *B. megaterium* AAK00598 amylase. The signal sequence is in shown bold.
SEQ ID NO: 28 is the amino acid sequence of AmyDSM90 amylase having the ΔRG deletion.
SEQ ID NO: 29 is the amino acid sequence of AmyDSM90 amylase having the M200L substitution.
SEQ ID NO: 30 is the amino acid sequence of AmyDSM90 amylase having the ΔRG deletion and the M200L substitution.

### DETAILED DESCRIPTION

Described are compositions and methods as defined in claims 1-16 relating to an α-amylase isolated from *Bacillus megaterium* strain DSM90, and to structurally related amylases. These amylases are collectively referred to as AmyDSM90-related amylases or AmyDSM90-related polypeptides. AmyDSM90 is a heretofore undescribed secreted amylase that has several unique structural features that distinguish it from related amylases. Moreover, the discovery that AmyDSM90-related polypeptides are secreted, rather than cytoplasmic polypeptides, enables the large scale expression and purification of various AmyDSM90-related polypeptides for industrial and commercial uses. Exemplary uses of these amylases are in the preparation of cleaning compositions, such as detergent compositions for cleaning laundry, dishes, textiles, and other surfaces. These and other aspects of the compositions and methods are described in detail, below.

### 1. Definitions and Acronyms

In accordance with this detailed description, the following abbreviations and definitions apply. Note that the singular forms "a," "an," and "the" include plural referents unless the context clearly dictates otherwise. Thus, for example, reference to "an enzyme" includes a plurality of such enzymes, and reference to "the dosage" includes reference to one or more dosages and equivalents thereof known to those skilled in the art, and so forth.

Unless defined otherwise, all technical and scientific terms used herein have the same meaning as commonly understood by one of ordinary skill in the art. The following abbreviations and/or terms are defined for clarity:

### 1.1 Abbreviations/acronyms

The following abbreviations/acronyms have the following meanings unless otherwise specified:
- AE: alcohol ethoxylate
- AEO: alcohol ethoxylate
- AEOS: alcohol ethoxysulfate
- AES: alcohol ethoxysulfate
- AmyDSM90: α-amylase from *Bacillus megaterium* strain DSM90
- AOS: α-olefinsulfonate
- AS: alkyl sulfate
- CBD-25: carbohydrate binding domain protein family 25
- cDNA: complementary DNA
- CMC: carboxymethylcellulose
- DNA: deoxyribonucleic acid
- DTMPA: diethylenetriaminepentaacetic acid
- EC: enzyme commission
- EDTA: ethylenediaminetetraacetic acid
- EMPA: Eidgenössische Materialprüfungs- und Forschungs Anstalt (Swiss Federal Laboratories for Materials Testing and Research)
- EO: ethylene oxide (polymer fragment)
- F&HC: fabric & household care
- GA: glucoamylase
- IPTG: isopropyl β-D-thiogalactoside
- kDa: kiloDalton
- LAS: linear alkylbenzenesulfonate
- LAT: *B. licheniformis* amylase
- MW: molecular weight
- MWU: modified Wohlgemuth unit; 1.6x10⁻⁵ mg/MWU = unit of activity
- NOBS: nonanoyloxybenzenesulfonate
- NTA: nitriloacetic acid
- OxAm: Purastar HPAM 5000L (Genencor International, Inc.)
- PEG: polyethyleneglycol
- pI: isoelectric point
- PVA: poly(vinyl alcohol)
- PVP: poly(vinylpyrrolidone)
- RNA: ribonucleic acid
- SAS: alkanesulfonate
- SDS-PAGE: sodium dodecyl sulfate polyacrylamide gel electrophoresis
- sp.: species,
- TAED: tetraacetylethylenediamine
- w/v: weight/volume
- w/w: weight/weight
- v/v: volume/volume
- wt%: weight percent
- °C: degrees Centigrade
- H₂O: water
- dH₂O or DI: deionized water
- dIH₂O: deionized water, Milli-Q filtration
- g or gm: grams
- µg: micrograms
- mg: milligrams
- kg: kilograms
- µL and µl: microliters
- mL and ml: milliliters
- mm: millimeters
- µm: micrometer
- M: molar
- mM: millimolar
- µM: micromolar
- U: units
- sec: seconds
- min(s): minute/minutes
- hr(s): hour/hours
- DO: dissolved oxygen
- Genencor: Danisco US Inc, Genencor Division, Palo Alto, CA
- Ncm: Newton centimeter
- ETOH: ethanol
- eq.: equivalents
- N: normal
- ds or DS: dry solids content

### 1.2 Definitions

The terms "amylase" or "amylolytic enzyme" refer to an enzyme that is, among other things, capable of catalyzing the degradation of starch. Amylases are hydrolases that cleave the α-D-(1→4) O-glycosidic linkages in starch. Generally, α-amylases (EC 3.2.1.1; α-D-(I→4)-glucan glucanohydrolase) are defined as endo-acting enzymes cleaving α-D-(1→4) O-glycosidic linkages within the starch molecule in a random fashion. In contrast, the exo-acting amylolytic enzymes, such as β-amylases (EC 3.2.1.2; α-D-(1→4)-glucan maltohydrolase) and some product-specific amylases like maltogenic α-amylase (EC 3.2.1.133) cleave the starch molecule from the non-reducing end of the substrate. β-Amylases, α-glucosidases (EC 3.2.1.20; α-D-glucoside glucohydrolase), glucoamylase (EC 3.2.1.3; α-D-(1→4)-glucan glucohydrolase), and product-specific amylases can produce malto-oligosaccharides of a specific length from starch.

As used herein the term "starch" refers to any material comprised of the complex polysaccharide carbohydrates of plants, comprised of amylose and amylopectin with the formula (C₆H₁₀O₅)ₓ, wherein X can be any number. The term includes plant-based materials such as grains, grasses, tubers and roots, and more specifically materials obtained from wheat, barley, corn, rye, rice, sorghum, brans, cassava, millet, potato, sweet potato, and tapioca.

The terms, "wild-type," "parental," or "reference," with respect to a polypeptide, refer to a naturally-occurring polypeptide that does not include a man-made substitution, insertion, or deletion at one or more amino acid positions. Similarly, the terms "wild-type," "parental," or "reference," with respect to a polynucleotide, refer to a naturally-occurring polynucleotide that does not include a man-made nucleoside change. However, note that a polynucleotide encoding a wild-type, parental, or reference polypeptide is not limited to a naturally-occurring polynucleotide, and encompasses any polynucleotide encoding the wild-type, parental, or reference polypeptide.

The term "variant," with respect to a polypeptide, refers to a polypeptide that differs from a specified wild-type, parental, or reference polypeptide in that it includes a man-made substitution, insertion, or deletion at one or more amino acid positions. Similarly, the term "variant," with respect to a polynucleotide, refers to a polynucleotide that differs in nucleotide sequence from a specified wild-type, parental, or reference polynucleotide. The identity of the wild-type, parental, or reference polypeptide or polynucleotide will be apparent from context.

The term "recombinant," when used in reference to a subject cell, nucleic acid, protein or vector, indicates that the subject has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell, or express native genes at different levels or under different conditions than found in nature.

The terms "recovered," "isolated," and "separated," refer to a compound, protein (polypeptides), cell, nucleic acid, amino acid, or other specified material or component that is removed from at least one other material or component with which it is naturally associated as found in nature.

As used herein, the term "purified" refers to material (*e.g*., an isolated polypeptide or polynucleotide) that is in a relatively pure state, *e.g*., at least about 90% pure, at least about 95% pure, at least about 98% pure, or even at least about 99% pure.

The terms "thermostable" and "thermostability," with reference to an enzyme, refer to the ability of the enzyme to retain activity after exposure to an elevated temperature. The thermostability of an enzyme, such as an amylase enzyme, is measured by its half-life (t_{1/2}) given in minutes, hours, or days, during which half the enzyme activity is lost under defined conditions. The half-life may be calculated by measuring residual α-amylase activity following exposure to (*i.e*., challenge by) an elevated temperature.

A "pH range," with reference to an enzyme, refers to the range of pH values under which the enzyme exhibits catalytic activity.

As used herein, the terms "pH stable" and "pH stability," with reference to an enzyme, relate to the ability of the enzyme to retain activity over a wide range of pH values for a predetermined period of time (*e.g*., 15 min., 30 min., 1 hour).

As used herein, the term "amino acid sequence" is synonymous with the terms "polypeptide," "protein," and "peptide," and are used interchangeably. Where such amino acid sequences exhibit activity, they may be referred to as an "enzyme." The conventional one-letter or three-letter codes for amino acid residues are used, with amino acid sequences being presented in the standard amino-to-carboxy terminal orientation (*i.e*., N→C).

The term "nucleic acid" encompasses DNA, RNA, heteroduplexes, and synthetic molecules capable of encoding a polypeptide. Nucleic acids may be single stranded or double stranded, and may be chemical modifications. The terms "nucleic acid" and "polynucleotide" are used interchangeably. Because the genetic code is degenerate, more than one codon may be used to encode a particular amino acid, and the present compositions and methods encompass nucleotide sequences that encode a particular amino acid sequence. Unless otherwise indicated, nucleic acid sequences are presented in 5'-to-3' orientation.

By "homologue" shall mean an entity having a specified degree of identity with the subject amino acid sequences and the subject nucleotide sequences. A homologous sequence is taken to include an amino acid sequence that is at least 75%, 80%, 85%, 86%, 87%, 88%, 89%, 90%, 91%, 92%, 93%, 94%, 95%, 96%, 97%, 98% or even 99% identical to the subject sequence, using conventional sequence alignment tools (*e.g.*, Clustal, BLAST, and the like). Typically, homologues will include the same active site residues as the subject amino acid sequence, unless otherwise specified.

As used herein, "hybridization" refers to the process by which one strand of nuclcic acid base pairs with a complementary strand, as occurs during blot hybridization techniques and PCR techniques. Stringent hybridization conditions are exemplified by the following: 50°C and 0.2X SSC (1X SSC = 0.15 M NaCl, 0.015 M Na₃ citrate, pH 7.0). Highly stringent conditions are exemplified by the following: 65°C and 0.1X SSC (1X SSC = 0.15 M NaCl, 0.015 M Na₃ citrate, pH 7.0)].

As used herein, a "synthetic" molecule is produced by *in vitro* chemical or enzymatic synthesis rather than by an organism.

As used herein, the terms "transformed," "stably transformed," and "transgenic," used with reference to a cell means that the cell contains a non-native (*e.g*., heterologous) nucleic acid sequence integrated into its genome or carried as an episome that is maintained through multiple generations.

The term "introduced" in the context of inserting a nucleic acid sequence into a cell, means "transfection", "transformation" or "transduction," as known in the art.

A "host strain" or "host cell" is an organism into which an expression vector, phage, virus, or other DNA construct, including a polynucleotide encoding a polypeptide of interest (*e.g*., a variant amylase) has been introduced. Exemplary host strains are bacterial cells. The term "host cell" includes protoplasts created from cells, such as those of a *Bacillus* sp.

The term "heterologous" with reference to a polynucleotide or protein refers to a polynucleotide or protein that does not naturally occur in a host cell.

The term "endogenous" with reference to a polynucleotide or protein refers to a polynucleotide or protein that occurs naturally in the host cell.

As used herein, the term "expression" refers to the process by which a polypeptide is produced based on a nucleic acid sequence. The process includes both transcription and translation.

A "selective marker" or "selectable marker" refers to a gene capable of being expressed in a host to facilitate selection of host cells carrying the gene. Examples of selectable markers include but are not limited to antimicrobials (*e.g.*, hygromycin, bleomycin, or chloramphenicol) and/or genes that confer a metabolic advantage, such as a nutritional advantage on the host cell.

A "vector" refers to a polynucleotide sequence designed to introduce nucleic acids into one or more cell types. Vectors include cloning vectors, expression vectors, shuttle vectors, plasmids, phage particles, cassettes and the like.

An "expression vector" refers to a DNA construct comprising a DNA sequence encoding a polypeptide of interest, which coding sequence is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences which control termination of transcription and translation.

The term "operably linked" means that specified components are in a relationship (including but not limited to juxtaposition) permitting them to function in an intended manner. For example, a regulatory sequence is operably linked to a coding sequence such that expression of the coding sequence is under control of the regulatory sequences.

A "signal sequence" is a sequence of amino acids attached to the N-terminal portion of a protein, which facilitates the secretion of the protein outside the cell. The mature form of an extracellular protein lacks the signal sequence, which is cleaved off during the secretion process.

As used herein, "biologically active" refer to a sequence having a specified biological activity, such an enzymatic activity.

"Water hardness" is a measure of the minerals (*e.g*., calcium and magnesium) present in water.

As used herein, "a cultured cell material comprising an AmyDSM90-related polypeptide," or similar language, refers to a cell lysate or supernatant (including media) that includes an AmyDSM90-related polypeptide as a component. The cell material is preferably from a heterologous host that is grown in culture for the purpose of producing the AmyDSM90-related polypeptide.

### 2. AmyDSM90-related Polypeptides and Nucleic Acids

One aspect of the present compositions and methods as defined in claims 1-16 is an AmyDSM90-related polypeptide. The polypeptide may correspond to AmyDSM90, an amylase having a specified degree of identity to AmyDSM90, variants of AmyDSM90that include man-made substitutions, insertions, or deletions, or chimeras, thereof. An exemplary AmyDSM90 polypeptide has the amino acid sequences of SEQ ID NO: 1. Additional polypeptides have at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or even 99% homology/identity to an AmyDSM90 polypeptide.

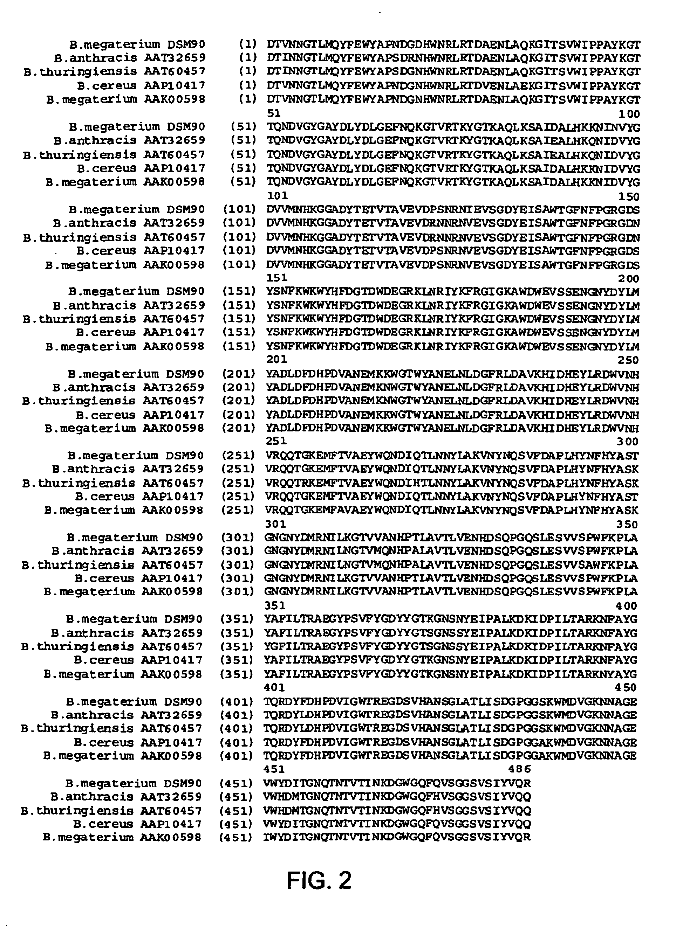

In some embodiments, the AmyDSM90 polypeptide has at least one of the following features (with respect to SEQ ID NO: 1): (a) an aspartic acid at position 21, (b) an asparagine at position 97, or (c) an isoleucine at position 128. The specified amino acid residues at these positions distinguish the AmyDSM90 polypeptides from orthologous amylases from *B*. *anthracis* AAT32659 (SEQ ID NO: 4), *B. thuringiensis* AAT60457 (SEQ ID NO: 5), *B*. *cereus* AAP10417 (SEQ ID NO: 6), and *B. megaterium* AAK00598 (SEQ ID NO: 3) (see, *e.g.,* Fig. 2). Notably, the presence of an aspartic acid at position 21 and an asparagine at position 97 in the AmyDSM90 polypeptide is in contrast to the converse arrangement observed in orthologous amylases (*i.e.,* the presence of an asparagine at position 21 aspartic acid at position 97). Without being limited to a theory, it is suggested that the presence of a positively charged amino acid residue at position 21 is normally important to enzyme activity, and that the AmyDSM90 polypeptide has overcome the need for this charge at position 21 by adopting a positively charged amino acid residue at position 97. However, this theory does not exclude the possibility that each of the above-identified features that distinguish the AmyDSM90 polypeptide from orthologous amylases are separate and independent.

Accordingly, in some embodiments, the AmyDSM90 polypeptides has only one of the above-identified features, *i.e*., an aspartic acid at position 21, an asparagine at position 97, or an isoleucine at position 128. In other embodiments, the α-amylase has two of the above-identified features, *i.e*., an aspartic acid at position 21 in combination with an asparagine at position 97, an aspartic acid at position 21 in combination with an isoleucine at position 128, or an asparagine at position 97 in combination with an isoleucine at position 128. In particular embodiments, the α-amylase has all three of the above-identified features, *i.e*., an aspartic acid at position 21, an asparagine at position 97, and an isoleucine at position 128.

The polypeptides may be an "immature" or "full-length" AmyDSM90-related polypeptide, which includes a signal sequence, or a mature form of an AmyDSM90-related polypeptides, which lacks a signal sequence. An exemplary immature form of the AmyDSM90 polypeptide has the amino acid sequences of SEQ ID NO: 2, while an exemplary mature form of the AmyDSM90 polypeptide has the amino acid sequences of SEQ ID NO: 1. Mature forms of the polypeptides are most useful for use in cleaning compositions. The polypeptides may also be a truncated form of an AmyDSM90-related polypeptide, which lacks the N or C-terminus of the mature form, or fragments of an AmyDSM90-related polypeptide that retains at least a portion of the α-amylase activity characteristic of the parental AmyDSM90-related polypeptide.

As noted above, the polypeptides include variant polypeptides, such as those that include man-made deletions, insertions, and substitutions. An exemplary deletion is the deletion of residues R179 and/or G180. An exemplary substitution is at residue M200, *e.g*., the M200L substitution. The R179-G180 deletion and M200 substitution can further be combined in a single polypeptide. Other exemplary substitutions are conservative amino acid substitutions, such as those listed in the following Table.

| *For Amino Acid* | *Code* | *Replace with any of* |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Orn, D-Om |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, b-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Om, D-Om |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4- carboxylic acid, D-or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

As mentioned, preferred polypeptides retain α-amylase activity but may have altered biochemical properties with reference to a naturally-occurring parental polypeptide. In some cases, the parental polypeptide is that of SEQ ID NO: 1.

The polypeptide may also be a chimeric polypeptide that includes at least a portion of an AmyDSM90-related polypeptide, and at least a portion of a second polypeptide. The second polypeptide may be, for example, a second amylase, a heterologous signal sequence, an epitope to allow tracking or purification, or the like. Exemplary heterologous signal sequences are from *B. subtilis* amylase (AmyE) or AprE, and *Streptomyces* CelA.

Another aspect of the present compositions and methods is a nucleic acid encoding an AmyDSM90-related polypeptide. The nucleic acid may encode AmyDSM90, an amylase having a specified degree of identity to AmyDSM90, variants of AmyDSM90 that include man-made substitutions, insertions, or deletions, or chimeras, thereof. In one examples, the nucleic acid encodes an amylase having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or even 99% homology/identity to an AmyDSM90 polypeptide, such as the polypeptide of SEQ ID NO: 1. The polypeptide may include at least one of the amino acid sequence features described above. It will be appreciated that due to the degeneracy of the genetic code, a plurality of nucleic acids may encode the same polypeptides.

The nucleic acid may also have a specified degree of homology to an exemplary polynucleotide encoding an AmyDSM90-related polypeptide, such as SEQ ID NO: 7, which encodes AmyDSM90. In one example, example, the nucleic acid has at least 75%, 80%, 85%, 86%, 87%, 88%, 89%, 90%, 91%, 92%, 93%, 94%, 95%, 96%, 97%, 98% or even 99% identity to the exemplary sequence. In another example, the nucleic acid hybridizes under stringent or very stringent conditions to the exemplary sequence.

Nucleic acids may encode a "full-length" ("fl" or "FL") AmyDSM90-related polypeptide, which includes a signal sequence, only the mature form of an AmyDSM90-related polypeptide, which lacks the signal sequence, a truncated form of AmyDSM90-related polypeptide, which lacks the N or C-terminus of the mature for, or fragments, thereof that retain at least a portion of the α-amylase activity characteristic of the AmyDSM90-related polypeptide.

A nucleic acid that encodes an AmyDSM90-related polypeptide can be operably linked to various promoters and regulators in a vector suitable for expressing an AmyDSM90-related polypeptide in host cells. Exemplary promoters are the *B. subtilis* Amy E and AprE promoters, and the *Streptomyces* CelA promoters. Such a nucleic acid can also be linked to other coding sequences, *e.g.*, to encode a chimeric polypeptide.

### 3. Method of Producing and Purifying Proteins

An aspect of the present compositions and method as defined in claims 1-16 is that AmyDSM90-related polypeptides can be expressed as secreted polypeptides. Notably, the orthologous α-amylases from *B. anthracis* AAT32659 (SEQ ID NO: 4), *B. thuringiensis* AAT60457 (SEQ ID NO: 5), and *B. cereus* AAP10417 (SEQ ID NO: 6) were heretofore believed to be cellular polypeptides, based on annotation in their Genbank entries. Cytoplasmic amylases are considered less valuable with respect to industrial and commercial processes because their production and recovery is more complicated. Therefore, the discovery that AmyDSM90-related polypeptides, including these orthologous α-amylases, are secreted greatly facilitates their isolation and purification, and enables their large scale use in industrial and commercial processes.

Methods of producing and purifying proteins that are secreted in to the culture medium from *Bacillus* are known in the art, as are suitable host cells for producing α-amylases. Exemplary methods for producing the α-amylases are disclosed below.

### 3.1 Materials and Methods for Producing α-Amylases

An AmyDSM90-related polypeptide can be expressed, in enzyme form, using an expression vector which typically includes control sequences encoding a suitable promoter, operator, ribosome binding site, translation initiation signal, and, optionally, a repressor gene or various activator genes. A large number of vectors are commercially available for use with recombinant DNA procedures, and the choice of vector will often depend on the host cell into which it is to be introduced. The vector may be an autonomously replicating vector, *i.e.*, a vector that exists as an extrachromosomal entity, the replication of which is independent of chromosomal replication, *e.g*., a plasmid, a bacteriophage or an extrachromosomal element, mini-chromosome or an artificial chromosome. Alternatively, the vector may be one which, when introduced into an isolated host cell, is integrated into the host cell genome and replicated together with the chromosome(s) into which it has been integrated. The integrated gene may also be amplified to create multiple copies of the gene in the chromosome by use of an amplifiable construct driven by antibiotic selection or other selective pressure, such as an essential regulatory gene or by complementation through dose effect of an essential metabolic pathway gene.

In the vector, the DNA sequence should be operably linked to a suitable promoter sequence. The promoter may be any DNA sequence that shows transcriptional activity in the host cell of choice and may be derived from genes encoding proteins either homologous or heterologous to the host cell. Exemplary promoters for directing the transcription of the DNA sequence encoding an AmyDSM90-related polypeptide, especially in a bacterial host, are the promoter of the lac operon of *E. coli*, the *Streptomyces coelicolor* agarase gene dagA or celA promoters, the promoters of the *Bacillus licheniformis* α-amylase gene (amyL), the promoters of the *Bacillus stearothermophilus* maltogenic amylase gene (amyM), the promoters of the *Bacillus amyloliquefaciens* α-amylase (amyQ), the promoters of the *Bacillus subtilis* xylA and xylB genes *etc.* For transcription in a fungal host, examples of useful promoters are those derived from the gene encoding *Aspergillus oryzae* TAKA amylase, *Rhizomucor miehei* aspartic proteinase, *Aspergillus nigger* neutral α-amylase, *A*. *niger* acid stable α-amylase, *A*. *nigger* glucoamylase, *Rhizomucor miehei* lipase, *A*. *oryzae* alkaline protease, *A. oryzae* triose phosphate isomerase, or *A. nidulans* acetamidase. When a gene encoding an AmyDSM90-related polypeptide is expressed in a bacterial species such as *E. coli*, a suitable promoter can be selected, for example, from a bacteriophage promoter including a T7 promoter and a phage lambda promoter. Examples of suitable promoters for the expression in a yeast species include but are not limited to the Gal 1 and Gal 10 promoters of *Saccharomyces cerevisiae* and the *Pichia pastoris* AOX1 or AOX2 promoters. For expression in *Trichoderma reesei*, the CBHII (cellobiohydrolase II) promoter may be used.

An expression vector may also comprise a suitable transcription terminator and, in eukaryotes, polyadenylation sequences operably linked to the DNA sequence encoding an AmyDSM90-related polypeptide. Termination and polyadenylation sequences may suitably be derived from the same sources as the promoter.

The vector may further comprise a DNA sequence enabling the vector to replicate in the host cell. Examples of such sequences are the origins of replication of plasmids pUC19, pACYC177, pUB110, pE194, pAMB1, and pIJ702.

The vector may also comprise a selectable marker, *e.g*., a gene the product of which complements a defect in the isolated host cell, such as the *dal* genes from *B. subtilis* or *B*. *licheniformis*, or a gene that confers antibiotic resistance such as, *e.g.*, ampicillin, kanamycin, chloramphenicol or tetracycline resistance. Furthermore, the vector may comprise *Aspergillus* selection markers such as *amdS, argB, niaD* and *xxsC*, a marker giving rise to hygromycin resistance, or the selection may be accomplished by co-transformation, such as known in the art. *See e.g.*, International PCT Application WO 91/17243.

As noted above, while intracellular expression or solid-state fermentation may be advantageous in some respects, *e.g*., when using certain bacteria or fungi as host cells, one aspect of the compositions and methods contemplates expression of an AmyDSM90-related polypeptide into the culture medium.

In general, the full-length or immature AmyDSM90-related polypeptides include a signal sequence at the amino terminus that permits secretion into the culture medium. If desirable, this signal peptide may be replaced by a different sequence, conveniently accomplished by substitution of the DNA sequences encoding the respective signal polypeptide.

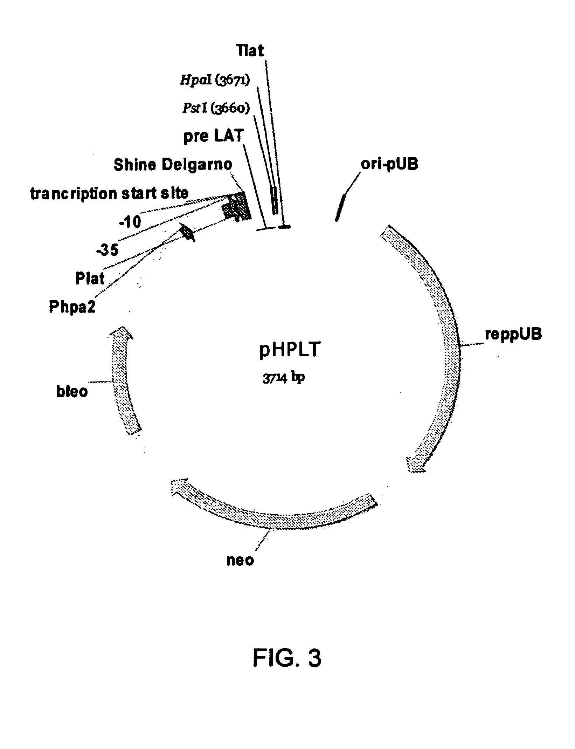

The expression vector typically includes the components of a cloning vector, such as, for example, an element that permits autonomous replication of the vector in the selected host organism and one or more phenotypically detectable markers for selection purposes. The expression vector normally comprises control nucleotide sequences such as a promoter, operator, ribosome binding site, translation initiation signal and optionally, a repressor gene or one or more activator genes. Additionally, the expression vector may comprise a sequence coding for an amino acid sequence capable of targeting the α-amylase variant to a host cell organelle such as a peroxisome, or to a particular host cell compartment. Such a targeting sequence includes but is not limited to the sequence, SKL. For expression under the direction of control sequences, the nucleic acid sequence of the α-amylase variant is operably linked to the control sequences in proper manner with respect to expression. A portion of an exemplary vector is depicted in FIG. 3.

The procedures used to ligate the DNA construct encoding an AmyDSM90-related polypeptide, the promoter, terminator and other elements, respectively, and to insert them into suitable vectors containing the information necessary for replication, are well known to persons skilled in the art (*see e.g.*, Sambrook et al., MOLECULAR CLONING: A LABORATORY MANUAL, 2nd ed., Cold Spring Harbor, 1989, and 3rd ed., 2001).

An isolated cell, either comprising a DNA construct or an expression vector, is advantageously used as a host cell in the recombinant production of an AmyDSM90-related polypeptide. The cell may be transformed with the DNA construct encoding the enzyme, conveniently by integrating the DNA construct (in one or more copies) in the host chromosome. This integration is generally considered to be an advantage, as the DNA sequence is more likely to be stably maintained in the cell. Integration of the DNA constructs into the host chromosome may be performed according to conventional methods, *e.g*., by homologous or heterologous recombination. Alternatively, the cell may be transformed with an expression vector as described above in connection with the different types of host cells.

Examples of suitable bacterial host organisms are Gram positive bacterial species such as *Bacillaceae* including *Bacillus subtilis, Bacillus Licheniformis, Bacillus lentus*, *Bacillus brevis, Geobacillus* (previously *Bacillus*) *stearothermophilus, Bacillus alkalophilus, Bacillus amyloliquefaciens, Bacillus coagulans, Bacillus lautus, Bacillus megaterium,* and *Bacillus thuringiensis; Streptomyces* species such as *Streptomyces murinus*; lactic acid bacterial species including *Lactococcus* sp. such as *Lactococcus lactis*; *Lactobacillus* sp. including *Lactobacillus reuteri; Leuconostoc* sp.; *Pediococcus* sp.; and *Streptococcus* sp. Alternatively, strains of a Gram negative bacterial species belonging to *Enterobacteriaceae* including *E. coli*, or to *Pseudomonadaceae* can be selected as the host organism.

A suitable yeast host organism can be selected from the biotechnologically relevant yeasts species such as but not limited to yeast species such as *Pichia* sp., *Hansenula* sp., or *Kluyveromyces, Yarrowinia*. *Schizosaccharomyces* species or a species of *Saccharomyces,* including *Saccharomyces cerevisiae* or a species belonging to *Schizosaccharomyces* such as, for example, *S. pombe* species. A strain of the methylotrophic yeast species, *Pichia pastoris,* can be used as the host organism. Alternatively, the host organism can be a *Hansenula* species. Suitable host organisms among filamentous fungi include species of *Aspergillus, e.g., Aspergillus niger, Aspergillus oryzae, Aspergillus tubigensis, Aspergillus awamori,* or *Aspergillus nidulans.* Alternatively, strains of a *Fusarium* species, *e.g., Fusarium oxysporum* or of a *Rhizomucor* species such as *Rhizomucor miehei* can be used as the host organism. Other suitable strains include *Thermomyces* and *Mucor* species. In addition, *Trichoderma reesei* can be used as a host. A suitable procedure for transformation of *Aspergillus* host cells includes, for example, that described in EP 238023.

In a yet further aspect, a method of producing an AmyDSM90-related polypeptide is provided comprising cultivating a host cell as described above under conditions conducive to the production of the enzyme and recovering the enzyme from the cells and/or culture medium.

The medium used to cultivate the cells may be any conventional medium suitable for growing the host cell in question and obtaining expression of an AmyDSM90-related polypeptide. Suitable media and media components are available from commercial suppliers or may be prepared according to published recipes (*e.g*., as described in catalogues of the American Type Culture Collection).

In one aspect, an enzyme secreted from the host cells is used in a whole broth preparation. In the present methods, the preparation of a spent whole fermentation broth of a recombinant microorganism can be achieved using any cultivation method known in the art resulting in the expression of an alpha-amylase. Fermentation may, therefore, be understood as comprising shake flask cultivation, small- or large-scale fermentation (including continuous, batch, fed-batch, or solid state fermentations) in laboratory or industrial fermenters performed in a suitable medium and under conditions allowing the amylase to be expressed or isolated. The term "spent whole fermentation broth" is defined herein as unfractionated contents of fermentation material that includes culture medium, extracellular proteins (*e.g.*, enzymes), and cellular biomass. It is understood that the term "spent whole fermentation broth" also encompasses cellular biomass that has been lysed or permeabilized using methods well known in the art.

An enzyme secreted from the host cells may conveniently be recovered from the culture medium by well-known procedures, including separating the cells from the medium by centrifugation or filtration, and precipitating proteinaceous components of the medium by means of a salt such as ammonium sulfate, followed by the use of chromatographic procedures such as ion exchange chromatography, affinity chromatography, or the like.

An aspect contemplates the polynucleotide in a vector is operably linked to a control sequence that is capable of providing for the expression of the coding sequence by the host cell, *i.e.* the vector is an expression vector. The control sequences may be modified, for example by the addition of further transcriptional regulatory elements to make the level of transcription directed by the control sequences more responsive to transcriptional modulators. The control sequences may in particular comprise promoters.

Host cells may be cultured under suitable conditions that allow expression of an AmyDSM90-related polypeptide. Expression of the enzymes may be constitutive such that they are continually produced, or inducible, requiring a stimulus to initiate expression. In the case of inducible expression, protein production can be initiated when required by, for example, addition of an inducer substance to the culture medium, for example dexamethasone or IPTG or Sopharose. Polypeptides can also be produced recombinantly in an *in vitro* cell-free system, such as the TNT™ (Promega) rabbit reticulocyte system.

An AmyDSM90-related polypeptide-expressing host also can be cultured in the appropriate medium for the host, under aerobic conditions. Shaking or a combination of agitation and aeration can be provided, with production occurring at the appropriate temperature for that host, *e.g.*, from about 25°C to about 75°C (*e.g.*, 30°C to 45°C), depending on the needs of the host and production of the desired α-amylase variant. Culturing can occur from about 12 to about 100 hours or greater (and any hour value there between, e.g., from 24 to 72 hours). Typically, the culture broth is at a pH of about 5.5 to about 8.0, again depending on the culture conditions needed for the host relative to production of an AmyDSM90-related polypeptide.

### 3.2 Materials and Methods for Protein Purification

Fermentation, separation, and concentration techniques are well known in the art and conventional methods can be used in order to prepare a concentrated AmyDSM90-related polypeptide-containing solution.

After fermentation, a fermentation broth is obtained, the microbial cells and various suspended solids, including residual raw fermentation materials, are removed by conventional separation techniques in order to obtain an amylase solution. Filtration, centrifugation, microfiltration, rotary vacuum drum filtration, ultrafiltration, centrifugation followed by ultrafiltration, extraction, or chromatography, or the like, are generally used.

It is desirable to concentrate an AmyDSM90-related polypeptide-containing solution in order to optimize recovery. Use of unconcentrated solutions requires increased incubation time in order to collect the purified enzyme precipitate.

The enzyme containing solution is concentrated using conventional concentration techniques until the desired enzyme level is obtained. Concentration of the enzyme containing solution may be achieved by any of the techniques discussed herein. Exemplary methods of purification include but are not limited to rotary vacuum filtration and/or ultrafiltration.

The enzyme solution is concentrated into a concentrated enzyme solution until the enzyme activity of the concentrated AmyDSM90-related polypeptide-containing solution is at a desired level.

Concentration may be performed using *e.g*., a precipitation agent, such as a metal halide precipitation agent. Metal halide precipitation agents include but are not limited to alkali metal chlorides, alkali metal bromides and blends of two or more of these metal halides. Exemplary metal halides include sodium chloride, potassium chloride, sodium bromide, potassium bromide and blends of two or more of these metal halides. The metal halide precipitation agent, sodium chloride, can also be used as a preservative.

The metal halide precipitation agent is used in an amount effective to precipitate the AmyDSM90-related polypeptide. The selection of at least an effective amount and an optimum amount of metal halide effective to cause precipitation of the enzyme, as well as the conditions of the precipitation for maximum recovery including incubation time, pH, temperature and concentration of enzyme, will be readily apparent to one of ordinary skill in the art, after routine testing.

Generally, at least about 5% w/v (weight/volume) to about 25% w/v of metal halide is added to the concentrated enzyme solution, and usually at least 8% w/v. Generally, no more than about 25% w/v of metal halide is added to the concentrated enzyme solution and usually no more than about 20% w/v. The optimal concentration of the metal halide precipitation agent will depend, among others, on the nature of the specific AmyDSM90-related polypeptide and on its concentration in the concentrated enzyme solution.

Another alternative to effect precipitation of the enzyme is to use organic compounds. Exemplary organic compound precipitating agents include: 4-hydroxybenzoic acid, alkali metal salts of 4-hydroxybenzoic acid, alkyl esters of 4-hydroxybenzoic acid, and blends of two or more of these organic compounds. The addition of said organic compound precipitation agents can take place prior to, simultaneously with or subsequent to the addition of the metal halide precipitation agent, and the addition of both precipitation agents, organic compound and metal halide, may be carried out sequentially or simultaneously.

Generally, the organic precipitation agents are selected from the group consisting of alkali metal salts of 4-hydroxybenzoic acid, such as sodium or potassium salts, and linear or branched alkyl esters of 4-hydroxybenzoic acid, wherein the alkyl group contains from 1 to 12 carbon atoms, and blends of two or more of these organic compounds. The organic compound precipitation agents can be, for example, linear or branched alkyl esters of 4-hydroxybenzoic acid, wherein the alkyl group contains from 1 to 10 carbon atoms, and blends of two or more of these organic compounds. Exemplary organic compounds are linear alkyl esters of 4-hydroxybenzoic acid, wherein the alkyl group contains from 1 to 6 carbon atoms, and blends of two or more of these organic compounds. Methyl esters of 4-hydroxybenzoic acid, propyl esters of 4-hydroxybenzoic acid, butyl ester of 4-hydroxybenzoic acid, ethyl ester of 4-hydroxybenzoic acid and blends of two or more of these organic compounds can also be used. Additional organic compounds also include but are not limited to 4-hydroxybenzoic acid methyl ester (named methyl PARABEN), 4-hydroxybenzoic acid propyl ester (named propyl PARABEN), which also are both amylase preservative agents. For further descriptions, *see*, *e.g.*, U.S. Patent No. 5,281,526.

Addition of the organic compound precipitation agent provides the advantage of high flexibility of the precipitation conditions with respect to pH, temperature, AmyDSM90-related polypeptide concentration, precipitation agent concentration, and time of incubation.

The organic compound precipitation agent is used in an amount effective to improve precipitation of the enzyme by means of the metal halide precipitation agent. The selection of at least an effective amount and an optimum amount of organic compound precipitation agent, as well as the conditions of the precipitation for maximum recovery including incubation time, pH, temperature and concentration of enzyme, will be readily apparent to one of ordinary skill in the art, in light of the present disclosure, after routine testing.

Generally, at least about 0.01% w/v of organic compound precipitation agent is added to the concentrated enzyme solution and usually at least about 0.02% w/v. Generally, no more than about 0.3% w/v of organic compound precipitation agent is added to the concentrated enzyme solution and usually no more than about 0.2% w/v.

The concentrated AmyDSM90-related polypeptides solution, containing the metal halide precipitation agent, and the organic compound precipitation agent, can he adjusted to a pH, which will, of necessity, depend on the enzyme to be purified. Generally, the pH is adjusted at a level near the isoelectric point of the amylase. The pH can be adjusted at a pH in a range from about 2.5 pH units below the isoelectric point (pI) up to about 2.5 pH units above the isoelectric point.

The incubation time necessary to obtain a purified enzyme precipitate depends on the nature of the specific enzyme, the concentration of enzyme, and the specific precipitation agent(s) and its (their) concentration. Generally, the time effective to precipitate the enzyme is between about 1 to about 30 hours; usually it does not exceed about 25 hours. In the presence of the organic compound precipitation agent, the time of incubation can still be reduced to less about 10 hours and in most cases even about 6 hours.

Generally, the temperature during incubation is between about 4°C and about 50°C. Usually, the method is carried out at a temperature between about 10°C and about 45°C (*e.g*., between about 20°C and about 40°C). The optimal temperature for inducing precipitation varies according to the solution conditions and the enzyme or precipitation agent(s) used.

The overall recovery of purified enzyme precipitate, and the efficiency with which the process is conducted, is improved by agitating the solution comprising the enzyme, the added metal halide and the added organic compound. The agitation step is done both during addition of the metal halide and the organic compound, and during the subsequent incubation period. Suitable agitation methods include mechanical stirring or shaking, vigorous aeration, or any similar technique.

After the incubation period, the purified enzyme is then separated from the dissociated pigment and other impurities and collected by conventional separation techniques, such as filtration, centrifugation, microfiltration, rotary vacuum filtration, ultrafiltration, press filtration, cross membrane microfiltration, cross flow membrane microfiltration, or the like. Further purification of the purified enzyme precipitate can be obtained by washing the precipitate with water. For example, the purified enzyme precipitate is washed with water containing the metal halide precipitation agent, or with water containing the metal halide and the organic compound precipitation agents.

During fermentation, an AmyDSM90-related polypeptide accumulates in the culture broth. For the isolation and purification of the desired AmyDSM90-related polypeptide, the culture broth is centrifuged or filtered to eliminate cells, and the resulting cell-free liquid is used for enzyme purification. In one embodiment, the cell-free broth is subjected to salting out using ammonium sulfate at about 70% saturation; the 70% saturation-precipitation fraction is then dissolved in a buffer and applied to a column such as a Sephadex G-100 column, and eluted to recover the enzyme-active fraction. For further purification, a conventional procedure such as ion exchange chromatography may be used.

Purified enzymes are useful for laundry and cleaning applications. For example, they can be used in laundry detergents and spot removers. They can be made into a final product that is either liquid (solution, slurry) or solid (granular, powder).

A more specific example of purification, is described in J. Sumitani et al., "New type of starch-binding domain: the direct repeat motif in the C-terminal region of Bacillus sp. no. 195 α-amylase contributes to starch binding and raw starch degrading," Biochem. J. 350: 477-484 (2000) and is briefly summarized here. The enzyme obtained from 4 liters of a *Streptomyces lividans* TK24 culture supernatant was treated with (NH₄)₂SO₄ at 80% saturation. The precipitate was recovered by centrifugation at 10,000 x g (20 minutes and 4°C) and re-dissolved in 20 mM Tris/HCl buffer (pH 7.0) containing 5 mM CaCl₂. The solubilized precipitate was then dialyzed against the same buffer. The dialyzed sample was then applied to a Sephacryl S-200 column, which had previously been equilibrated with 20 mM Tris/HCl buffer, (pH 7.0), 5 mM CaCl₂, and eluted at a linear flow rate of 7 mL/hr with the same buffer. Fractions from the column were collected and assessed for activity as judged by enzyme assay and SDS-PAGE. The protein was further purified as follows. A Toyopearl HW55 column (Tosoh Bioscience, Montgomeryville, PA; Cat. No. 19812) was equilibrated with 20 mM Tris/HCl buffer (pH 7.0) containing 5 mM CaCl₂ and 1.5 M (NH₄)₂SO₄. The enzyme was eluted with a linear gradient of 1.5 to 0 M (NH₄)₂SO₄ in 20 mM Tris/HCL buffer, pH 7.0 containing 5 mM CaCl₂. The active fractions were collected, and the enzyme precipitated with (NH₄)₂SO₄ at 80% saturation. The precipitate was recovered, re-dissolved, and dialyzed as described above. The dialyzed sample was then applied to a Mono Q HR5/5 column (Amersham Pharmacia; Cat. No. 17-5167-01) previously equilibrated with 20 mM Tris/HCl buffer (pH 7.0) containing 5 mM CaCl₂, at a flow rate of 60 mL/hour. The active fractions are collected and added to a 1.5 M (NH₄)₂SO₄ solution. The active enzyme fractions were re-chromatographed on a Toyopearl HW55 column, as before, to yield a homogeneous enzyme as determined by SDS-PAGE. *See* J. Sumitani et al., "New type of starch-binding domain: the direct repeat motif in the C-terminal region of Bacillus sp. no. 195 α-amylase contributes to starch binding and raw starch degrading," Biochem. J. 350: 477-484 (2000) for general discussion of the method and variations thereon.

For production scale recovery, an AmyDSM90-related polypeptide can be partially purified as generally described above by removing cells via flocculation with polymers. Alternatively, the enzyme can be purified by microfiltration followed by concentration by ultrafiltration using available membrane and equipment. However, for some applications, the enzyme does not need to be purified, and whole broth culture can be lysed and used without further treatment. The enzyme can then be processed, for example, into granules.

### 4. Cleaning Compositions

An aspect of the present compositions and methods is a cleaning composition that includes an AmyDSM90-related polypeptide as a component. An AmyDSM90-related polypeptide can be used as a component in detergent compositions for hand washing, laundry washing, dishwashing, and other hard-surface cleaning. Preferably, an AmyDSM90-related polypeptide is incorporated into detergents at or near a concentration conventionally used for amylase in detergents. For example, an AmyDSM90-related polypeptide may be added in amount corresponding to 0.00001 - 1 mg (calculated as pure enzyme protein) of α-amylase per liter of wash/dishwash liquor. Exemplary formulations are provided herein, as exemplified by the following:

### 4.1 Laundry Detergent Composition

An AmyDSM90-related polypeptide may typically be a component of a detergent composition, as the only enzyme or with other enzymes including other amylolytic enzymes. As such, it may be included in the detergent composition in the form of a non-dusting granulate, a stabilized liquid, or a protected enzyme. Non-dusting granulates may be produced, *e.g*., as disclosed in U.S. Patent Nos. 4,106,991 and 4,661,452 and may optionally be coated by methods known in the art. Examples of waxy coating materials are poly(ethylene oxide) products (polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in, for example, GB Patent No. 1483591. Liquid enzyme preparations may, for instance, be stabilized by adding a polyol such as propylene glycol, a sugar or sugar alcohol, lactic acid or boric acid according to established methods. Other enzyme stabilizers are known in the art. Protected enzymes may be prepared according to the method disclosed in for example EP 238,216. Polyols have long been recognized as stabilizers of proteins, as well as improving protein solubility. *See, e.g.*, J. K. Kaushik et al., "Why is trehalose an exceptional protein stabilizer?" J. Biol. Chem. 278: 26458-65 (2003) and reference cited therein; and Monica Conti et al., "Capillary isoelectric focusing: the problem of protein solubility," J. Chromatography A 757: 237-245 (1997).

The detergent composition may be in any useful form, e.g., as powders, granules, pastes, or liquid. A liquid detergent may be aqueous, typically containing up to about 70% of water and 0% to about 30% of organic solvent. It may also be in the form of a compact gel type containing only about 30% water.

The detergent composition comprises one or more surfactants, each of which may be anionic, nonionic, cationic, or zwitterionic. The detergent will usually contain 0% to about 50% of anionic surfactant, such as linear alkylbenzenesulfonate (LAS); α-olefinsulfonate (AOS); alkyl sulfate (fatty alcohol sulfate) (AS); alcohol ethoxysulfate (AEOS or AES); secondary alkanesulfonates (SAS); α-sulfo fatty acid methyl esters; alkyl- or alkenylsuccinic acid; or soap. The composition may also contain 0% to about 40% of nonionic surfactant such as alcohol ethoxylate (AEO or AE), carboxylated alcohol ethoxylates, nonylphenol ethoxylate, alkylpolyglycoside, alkyldimethylamineoxide, ethoxylated fatty acid monoethanolamide, fatty acid monoethanolamide, or polyhydroxy alkyl fatty acid amide (as described for example in WO 92/06154).

The detergent composition may additionally comprise one or more other enzymes, such as lipase, another amylolytic enzyme, cutinase, protease, cellulase, peroxidase, and/or laccase in any combination.

The detergent may contain about 1% to about 65% of a detergent builder or complexing agent such as zeolite, diphosphate, triphosphate, phosphonate, citrate, nitrilotriacetic acid (NTA), ethylenediaminetetraacetic acid (EDTA), diethylenetriaminepentaacetic acid (DTMPA), alkyl- or alkenylsuccinic acid, soluble silicates or layered silicates (*e.g.*, SKS-6 from Hoechst). The detergent may also be unbuilt, *i.e*. essentially free of detergent builder. The enzymes can be used in any composition compatible with the stability of the enzyme. Enzymes generally can be protected against deleterious components by known forms of encapsulation, for example, by granulation or sequestration in hydro gels. Enzymes, and specifically α-amylases, such as AmyDSM90 molecules, either with or without starch binding domains, can be used in a variety of compositions including laundry and dishwashing applications, surface cleaners, as well as in compositions for ethanol production from starch or biomass.

The detergent may comprise one or more polymers. Examples include carboxymethylcellulose (CMC), poly(vinylpyrrolidone) (PVP), polyethyleneglycol (PEG), poly(vinyl alcohol) (PVA), polycarboxylates such as polyacrylates, maleic/acrylic acid copolymers and lauryl methacrylate/acrylic acid copolymers.

The detergent may contain a bleaching system, which may comprise a H₂O₂ source such as perborate or percarbonate, which may be combined with a peracid-forming bleach activator such as tetraacetylethylenediamine (TAED) or nonanoyloxybenzenesulfonate (NOBS). Alternatively, the bleaching system may comprise peroxyacids (*e.g*., the amide, imide, or sulfone type peroxyacids). The bleaching system can also be an enzymatic bleaching system, for example, perhydrolase, such as that described in International PCT Application WO 2005/056783.

The enzymes of the detergent composition may be stabilized using conventional stabilizing agents, *e.g*., a polyol such as propylene glycol or glycerol; a sugar or sugar alcohol; lactic acid; boric acid or a boric acid derivative such as, *e.g*., an aromatic borate ester; and the composition may be formulated as described in, *e.g*., WO 92/19709 and WO 92/19708.

The detergent may also contain other conventional detergent ingredients such as *e.g*., fabric conditioners including clays, foam boosters, suds suppressors, anti-corrosion agents, soilsuspending agents, anti-soil redeposition agents, dyes, bactericides, tarnish inhibiters, optical brighteners, or perfumes.

The pH (measured in aqueous solution at use concentration) is usually neutral or alkaline, *e.g*., pH about 7.0 to about 11.0.

Particular forms of detergent compositions comprising the AmyDSM90-related polypeptide can be formulated to include:
1) A detergent composition formulated as a granulate having a bulk density of at least 600 g/L comprising linear alkylbenzenesulfonate (calculated as acid) about 7% to about 12%; alcohol ethoxysulfate (*e.g*., C₁₂₋₁₈ alcohol, 1-2 ethylene oxide (EO)) or alkyl sulfate (*e.g.*, C₁₆₋₁₈) about 1% to about 4%; alcohol ethoxylate (*e.g*., C₁₄₋₁₅ alcohol, 7 EO) about 5% to about 9%; sodium carbonate (*e.g*., Na₂CO₃) about 14% to about 20%; soluble silicate (*e.g.,* Na₂O, 2SiO₂) about 2 to about 6%; zeolite (*e.g*., NaAlSiO₄) about 15% to about 22%; sodium sulfate (*e.g.*, Na₂SO₄) 0% to about 6%; sodium citrate/citric acid (*e.g*., C₆H₅Na₃O₇/C₆H₈O₇) about 0% to about 15%; sodium perborate (*e.g*., NaBO₃H₂O) about 11% to about 18%; TAED about 2% to about 6%; carboxymethylcellulose (CMC) and 0% to about 2%; polymers (*e.g*., maleic/acrylic acid, copolymer, PVP, PEG) 0-3%; enzymes (calculated as pure enzyme) 0.0001-0.1% protein; and minor ingredients (*e.g.*, suds suppressors, perfumes, optical brightener, photobleach) 0-5%.
2) A detergent composition formulated as a granulate having a hulk density of at least 600 g/L comprising linear alkylbenzenesulfonate (calculated as acid) about 6% to about 11%; alcohol ethoxysulfate (e.g., C₁₂₋₁₈ alcohol, 1-2 EO) or alkyl sulfate (*e.g.*, C₁₆₋₁₈) about 1% to about 3%; alcohol ethoxylate (*e.g.*, C₁₄₋₁₅ alcohol, 7 EO) about 5% to about 9%; sodium carbonate (*e.g*., Na₂CO₃) about 15% to about 21%; soluble silicate (*e.g.,* Na₂O, 2SiO₂) about 1% to about 4%; zeolite (*e.g.*, NaAlSiO₄) about 24% to about 34%; sodium sulfate (*e.g.*, Na₂SO₄) about 4% to about 10%; sodium citrate/citric acid (*e.g.*, C₆H₅Na₃O₇/ C₆H₈O₇) 0% to about 15%; carboxymethylcellulose (CMC) 0% to about 2%; polymers (e.g., maleic/acrylic acid copolymer, PVP, PEG) 1-6%; enzymes (calculated as pure enzyme protein) 0.0001-0.1%; minor ingredients (*e.g*., suds suppressors, perfume) 0-5%.
3) A detergent composition formulated as a granulate having a bulk density of at least 600 g/L comprising linear alkylbenzenesulfonate (calculated as acid) about 5% to about 9%; alcohol ethoxylate (*e.g*., C₁₂₋₁₅ alcohol, 7 EO) about 7% to about 14%; Soap as fatty acid (*e.g*., C₁₆₋₂₂ fatty acid) about 1 to about 3%; sodium carbonate (as Na₂CO₃) about 10% to about 17%; soluble silicate (*e.g.*, Na₂O, 2SiO₂) about 3% to about 9%; zeolite (as NaAlSiO₄) about 23% to about 33%; sodium sulfate (*e.g*., Na₂SO₄) 0% to about 4%; sodium perborate (*e.g*., NaBO₃H₂O) about 8% to about 16%; TAED about 2% to about 8%; phosphonate (*e.g*., EDTMPA) 0% to about 1%; carboxymethylcellulose (CMC) 0% to about 2%; polymers (*e.g*., maleic/acrylic acid copolymer, PVP, PEG) 0-3%; enzymes (calculated as pure enzyme protein) 0.0001-0.1%; minor ingredients (*e.g*., suds suppressors, perfume, optical brightener) 0-5%.
4) A detergent composition formulated as a granulate having a bulk density of at least 600 g/L comprising linear alkylbenzenesulfonate (calculated as acid) about 8% to about 12%; alcohol ethoxylate (*e.g*., C₁₂₋₁₅ alcohol, 7 EO) about 10% to about 25%; sodium carbonate (as Na₂CO₃) about 14% to about 22%; soluble silicate (*e.g.*, Na₂O, 2SiO₂) about 1% to about 5%; zeolite (*e.g.*, NaAlSiO₄) about 25% to about 35%; sodium sulfate (*e.g.*, Na₂SO₄) 0% to about 10%; carboxymethylcellulose (CMC) 0% to about 2%; polymers (*e.g*., maleic/acrylic acid copolymer, PVP, PEG) 1-3%; enzymes (calculated as pure enzyme protein) 0.0001-0.1%; and minor ingredients (*e.g*., suds suppressors, perfume) 0-5%.
5) An aqueous liquid detergent composition comprising linear alkylbenzenesulfonate (calculated as acid) about 15% to about 21%; alcohol ethoxylate (*e.g*., C₁₂₋₁₅ alcohol, 7 EO or C₁₂₋₁₅ alcohol, 5 EO) about 12% to about 18%; soap as fatty acid (*e.g.*, oleic acid) about 3% to about 13%; alkenylsuccinic acid (C₁₂₋₁₄) 0% to about 13%; aminoethanol about 8% to about 18%; citric acid about 2% to about 8%; phosphonate 0% to about 3%; polymers (*e.g*., PVP, PEG) 0% to about 3%; borate (*e.g*., B₄O₇) 0% to about 2%; ethanol 0% to about 3%; propylene glycol about 8% to about 14%; enzymes (calculated as pure enzyme protein) 0.0001-0.1 %; and minor ingredients (*e.g*., dispersants, suds suppressors, perfume, optical brightener) 0-5%.
6) An aqueous structured liquid detergent composition comprising linear alkylbenzenesulfonate (calculated as acid) about 15% to about 21%; alcohol ethoxylate (*e.g*., C₁₂₋₁₅ alcohol, 7 EO, or C₁₂₋₁₅ alcohol, 5 EO) 3-9%; soap as fatty acid (*e.g*., oleic acid) about 3% to about 10%; zeolite (as NaAlSiO₄) about 14% to about 22%; potassium citrate about 9% to about 18%; borate (*e.g*., B₄O₇) 0% to about 2%; carboxymethylcellulose (CMC) 0% to about 2%; polymers (*e.g*., PEG, PVP) 0% to about 3%; anchoring polymers such as, *e.g.*, lauryl methacrylate/acrylic acid copolymer; molar ratio 25:1, MW 3800) 0% to about 3%;glycerol 0% to about 5%; enzymes (calculated as pure enzyme protein) 0.0001-0.1%; and minor ingredients (*e.g*., dispersants, suds suppressors, perfume, optical brighteners) 0-5%.
7) A detergent composition formulated as a granulate having a bulk density of at least 600 g/L comprising fatty alcohol sulfate about 5% to about 10%; ethoxylated fatty acid monoethanolamide about 3% to about 9%; soap as fatty acid 0-3%; sodium carbonate (*e.g*., Na₂CO₃) about 5% to about 10%; Soluble silicate (*e.g*., Na₂O, 2SiO₂) about 1% to about 4%; zeolite (*e.g.*, NaAlSiO₄) about 20% to about 40%; Sodium sulfate (*e.g*., Na₂SO₄) about 2% to about 8%; sodium perborate (*e.g*., NaBO₃H₂O) about 12% to about 18%; TAED about 2% to about 7%; polymers (*e.g*., maleic/acrylic acid copolymer, PEG) about 1% to about 5%; enzymes (calculated as pure enzyme protein) 0.0001-0.1%; and minor ingredients (*e.g*., optical brightener, suds suppressors, perfume) 0-5%.
8) A detergent composition formulated as a granulate comprising linear alkylbenzenesulfonate (calculated as acid) about 8% to about 14%; ethoxylated fatty acid monoethanolamide about 5% to about 11%; soap as fatty acid 0% to about 3%; sodium carbonate (*e.g*., Na₂CO₃) about 4% to about 10%; soluble silicate (Na₂O, 2SiO₂) about 1% to about 4%; zeolite (*e.g*., NaAlSiO₄) about 30% to about 50%; sodium sulfate (*e.g.*, Na₂SO₄) about 3% to about 11%; sodium citrate (*e.g*., C₆H₅Na₃O₇) about 5% to about 12%; polymers (*e.g*., PVP, maleic/acrylic acid copolymer, PEG) about 1% to about 5%; enzymes (calculated as pure enzyme protein) 0.0001-0.1%; and minor ingredients (*e.g*., suds suppressors, perfume) 0-5%.
9) A detergent composition formulated as a granulate comprising linear alkylbenzenesulfonate (calculated as acid) about 6% to about 12%; nonionic surfactant about 1% to about 4%; soap as fatty acid about 2% to about 6%; sodium carbonate (*e.g*., Na₂CO₃) about 14% to about 22%; zeolite (*e.g*., NaAlSiO₄) about 18% to about 32%; sodium sulfate (*e.g*., Na₂SO₄) about 5% to about 20%; sodium citrate (*e.g*., C₆H₅Na₃O₇) about 3% to about 8%; sodium perborate (*e.g*., NaBO₃H₂O) about 4% to about 9%; bleach activator (*e.g*., NOBS or TAED) about 1% to about 5%; carboxymethylcellulose (CMC) 0% to about 2%; polymers (*e.g*., polycarboxylate or PEG) about 1% to about 5%; enzymes (calculated as pure enzyme protein) 0.0001-0.1%; and minor ingredients (*e.g*., optical brightener, perfume) 0-5%.
10) An aqueous liquid detergent composition comprising linear alkylbenzenesulfonate (calculated as acid) about 15% to about 23%; alcohol ethoxysulfate (*e.g*., C₁₂₋₁₅ alcohol, 2-3 EO) about 8% to about 15%; alcohol ethoxylate (*e.g*., C₁₂₋₁₅ alcohol, 7 EO, or C₁₂₋₁₅ alcohol, 5 EO) about 3% to about 9%; soap as fatty acid (*e.g*., lauric acid) 0% to about 3%; aminoethanol about 1% to about 5%; sodium citrate about 5% to about 10%; hydrotrope (*e.g*., sodium toluensulfonate) about 2% to about 6%; borate (*e.g*., B₄O₇) 0% to about 2%; carboxymethylcellulose 0% to about 1%; ethanol about 1% to about 3%; propylene glycol about 2% to about 5%; enzymes (calculated as pure enzyme protein) 0.0001-0.1%; and minor ingredients (*e.g*., polymers, dispersants, perfume, optical brighteners) 0-5%.
11) An aqueous liquid detergent composition comprising linear alkylbenzenesulfonate (calculated as acid) about 20% to about 32%; alcohol ethoxylate (*e.g*., C₁₂₋₁₅ alcohol, 7 EO, or C₁₂₋₁₅ alcohol, 5 EO) 6-12%; aminoethanol about 2% to about 6%; citric acid about 8% to about 14%; borate (*e.g*., B₄O₇) about 1% to about 3%; polymer (*e.g*., maleic/acrylic acid copolymer, anchoring polymer such as, *e.g*., lauryl methacrylate/acrylic acid copolymer) 0% to about 3%; glycerol about 3% to about 8%; enzymes (calculated as pure enzyme protein) 0.0001-0.1%; and minor ingredients (*e.g*., hydrotropes, dispersants, perfume, optical brighteners) 0-5%.
12) A detergent composition formulated as a granulate having a bulk density of at least 600 g/L comprising anionic surfactant (linear alkylbenzenesulfonate, alkyl sulfate, α-olefinsulfonate, α-sulfo fatty acid methyl esters, alkanesulfonates, soap) about 25% to about 40%; nonionic surfactant (*e.g*., alcohol ethoxylate) about 1% to about 10%; sodium carbonate *(e.g.,* Na₂CO₃) about 8% to about 25%; soluble silicates (*e.g*., Na₂O, 2SiO₂) about 5% to about 15%; sodium sulfate (*e.g*., Na₂SO₄) 0% to about 5%; zeolite (NaAlSiO₄) about 15% to about 28%; sodium perborate (*e.g*., NaBO₃·4H₂O) 0% to about 20%; bleach activator (TAED or NOBS) about 0% to about 5%; enzymes (calculated as pure enzyme protein) 0.0001-0.1 %; minor ingredients (*e.g*., perfume, optical brighteners) 0-3%.
13) Detergent compositions as described in compositions 1)-12) *supra*, wherein all or part of the linear alkylbenzenesulfonate is replaced by (C₁₂₋₁₈) alkyl sulfate.
14) A detergent composition formulated as a granulate having a bulk density of at least 600 g/L comprising (C₁₂-C₁₈) alkyl sulfate about 9% to about 15%; alcohol ethoxylate about 3% to about 6%; polyhydroxy alkyl fatty acid amide about 1% to about 5%; zeolite (*e.g*., NaAlSiO₄) about 10% to about 20%; layered disilicate (*e.g*., SK56 from Hoechst) about 10% to about 20%; sodium carbonate (*e.g*., Na₂CO₃) about 3% to about 12%; soluble silicate (*e.g*., Na₂O, 2SiO₂) 0% to about 6%; sodium citrate about 4% to about 8%; sodium percarbonate about 13% to about 22%; TAED about 3% to about 8%; polymers (*e.g*., polycarboxylates and PVP) 0% to about 5%; enzymes (calculated as pure enzyme protein) 0.0001-0.1%; and minor ingredients (*e.g*., optical brightener, photobleach, perfume, suds suppressors) 0-5%.
15) A detergent composition formulated as a granulate having a bulk density of at least 600 g/L comprising (C₁₂-C₁₈) alkyl sulfate about 4% to about 8%; alcohol ethoxylate about 11% to about 15%; soap about 1% to about 4%; zeolite MAP or zeolite A about 35% to about 45%; sodium carbonate (as Na₂CO₃) about 2% to about 8%; soluble silicate (*e.g*., Na₂O, 2SiO₂) 0% to about 4%; sodium percarbonate about 13% to about 22%; TAED 1-8%; carboxymethylcellulose (CMC) 0% to about 3%; polymers (*e.g*., polycarboxylates and PVP) 0% to about 3%; enzymes (calculated as pure enzyme protein) 0.0001-0.1%; and minor ingredients (*e.g*., optical brightener, phosphonate, perfume) 0-3%.
16) Detergent formulations as described in 1)-15) supra, which contain a stabilized or encapsulated peracid, either as an additional component or as a substitute for already specified bleach systems.
17) Detergent compositions as described supra in 1), 3), 7), 9), and 12), wherein perborate is replaced by percarbonate.
18) Detergent compositions as described supra in 1), 3), 7), 9), 12), 14), and 15), which additionally contain a manganese catalyst. The manganese catalyst for example is one of the compounds described in "Efficient manganese catalysts for low-temperature bleaching," Nature 369: 637-639 (1994).
19) Detergent composition formulated as a non-aqueous detergent liquid comprising a liquid nonionic surfactant such as, *e.g*., linear alkoxylated primary alcohol, a builder system (*e.g*., phosphate), an enzyme(s), and alkali. The detergent may also comprise anionic surfactant and/or a bleach system.

An AmyDSM90-related polypeptide may be incorporated at a concentration conventionally employed in detergents. It is at present contemplated that, in the detergent composition, the enzyme may be added in an amount corresponding to 0.00001-1.0 mg (calculated as pure enzyme protein) of AmyDSM90-related polypeptide per liter of wash liquor.

In another embodiment, other enzymes, such as 2,6-β-D-fructan hydrolase, can be incorporated in detergent compositions comprising an AmyDSM90-related polypeptide and used for removal/cleaning of biofilm present on household and/or industrial textile/laundry.

The detergent composition may for example be formulated as a hand (manual) or machine (automatic) laundry detergent composition, including a laundry additive composition suitable for pre-treatment of stained fabrics and a rinse added fabric softener composition, or be formulated as a detergent composition for use in general household hard surface cleaning operations, or be formulated for manual or automatic dishwashing operations.

In a specific aspect, the detergent composition can comprise 2,6-β-D-fructan hydrolase in addition to an AmyDSM90-related polypeptide, and one or more other cleaning enzymes, such as a protease, a lipase, a cutinase, a carbohydrase, a cellulase, a pectinase, a mannanase, an arabinase, a galactanase, another amylolytic enzyme, a xylanase, an oxidase, a laccase, and/or a peroxidase, and/or combinations thereof.

In general the properties of the chosen enzyme(s) should be compatible with the selected detergent, (*e.g*., pH-optimum, compatibility with other enzymatic and non-enzymatic ingredients, *etc*.), and the enzyme(s) should be present in effective amounts.

*Proteases*: Suitable proteases include those of animal, vegetable or microbial origin. Chemically modified or protein engineered mutants are included, as well as naturally processed proteins. The protease may be a serine protease or a metalloprotease, such as an alkaline microbial protease, a trypsin-like protease, or a chymotrypsin-like protease. Examples of alkaline proteases are subtilisins, especially those derived from *Bacillus, e.g*., subtilisin Novo, subtilisin Carlsberg, subtilisin 309, subtilisin 147, and subtilisin 168 (*see, e.g*., WO 89/06279). Examples of trypsin-like proteases are trypsin (*e.g*., of porcine or bovine origin), and *Fusarium* proteases (*see, e.g*., WO 89/06270 and WO 94/25583). Examples of useful proteases also include but are not limited to the variants described in WO 92/19729, WO 98/20115, WO 98/20116, and WO 98/34946. Commercially available protease enzymes include but are not limited to: ALCALASE®, SAVINASE®, PRIMASE™, DURALASE™, ESPERASE®, and KANNASE™ (Novo Nordisk A/S); MAXATASE®, MAXACAL™, MAXAPEM™, PROPERASE®, PURAFECT®, PURAFECT OXP™, FN2™, and FN3™ (Genencor International, Inc.).

*Lipases*: Suitable lipases include those of bacterial or fungal origin. Chemically modified, proteolytically modified, or protein engineered mutants are included. Examples of useful lipases include but are not limited to lipases from *Humicola* (synonym *Thermomyces*), *e.g*., from *H. lanuginosa* (*T. lanuginosus*) (*see e.g.,* EP 258068 and EP 305216), from H. *insolens* (*see e.g.,* WO 96/13580); a *Pseudomonas* lipase (*e.g*., from *P. alcaligenes* or *P. pseudoalcaligenes; see, e.g.,* EP 218 272), *P. cepacia* (*see e.g.,* EP 331 376), *P. stutzeri* (*see e.g.,* GB 1,372,034), *P. fluorescens, Pseudomonas* sp. strain SD 705 (*see e.g.,* WO 95/06720 and WO 96/27002), *P. wisconsinensis* (*see e.g.,* WO 96/12012); a *Bacillus* lipase (*e.g.,* from *B. subtilis*; *see e.g*., Dartois et al. Biochemica et Biophysica Acta, 1131: 253-360 (1993)), *B. stearothermophilus* (*see e.g.,* JP 64/744992), or *B. pumilus* (*see e.g.,* WO 91/16422). Additional lipase variants contemplated for use in the formulations include those described for example in: WO 92/05249, WO 94/01541, WO 95/35381, WO 96/00292, WO 95/30744, WO 94/25578, WO 95/14783, WO 95/22615, WO 97/04079, WO 97/07202, EP 407225, and EP 260105. Some commercially available lipase enzymes include LIPOLASE® and LIPOLASE ULTRA™ (Novo Nordisk A/S).

*Polyesterases*: Suitable polyesterases can be included in the composition, such as those described in, for example, WO 01/34899 and WO 01/14629.

Amylases: The compositions can be combined with other amylases, such as non-production enhanced α-amylase. These can include commercially available amylases, such as but not limited to DURAMYL®, TERMAMYL®, FUNGAMYL® and BAN™ (Novo Nordisk A/S); RAPIDASE® and PURASTAR® (from Genencor International, Inc.).

*Cellulases*: Cellulases can be added to the compositions. Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g*., the fungal cellulases produced from *Humicola insolens, Myceliophthora thermophila* and *Fusarium oxysporum* disclosed for example in U.S. Patent Nos. 4,435,307; 5,648,263; 5,691,178; 5,776,757; and WO 89/09259. Exemplary cellulases contemplated for use are those having color care benefit for the textile. Examples of such cellulases are cellulases described in for example EP 0495257, EP 0531372, WO 96/11262, WO 96/29397, and WO 98/08940. Other examples are cellulase variants, such as those described in WO 94/07998; WO 98/12307; WO 95/24471; PCT/DK98/00299; EP 531315; U.S. Patent Nos. 5,457,046; 5,686,593; and 5,763,254. Commercially available cellulases include CELLUZYME® and CAREZYME® (Novo Nordisk A/S); CLAZINASE® and PURADAX HA® (Genencor International, Inc.); and KAC-500(B)™ (Kao Corporation).

*Peroxidases*/*Oxidases*: Suitable peroxidases/oxidases contemplated for use in the compositions include those of plant, bacterial or fungal origin. Chemically modified or protein engineered mutants are included. Examples of useful peroxidases include peroxidases from *Coprims, e.g*., from *C. cinereus*, and variants thereof as those described in WO 93/24618, WO 95/10602, and WO 98/15257. Commercially available peroxidases include for example GUARDZYME™ (Novo Nordisk A/S).

The detergent enzyme(s) may be included in a detergent composition by adding separate additives containing one or more enzymes, or by adding a combined additive comprising all of these enzymes. A detergent additive, *i.e.* a separate additive or a combined additive, can be formulated *e.g*., as a granulate, a liquid, a slurry, and the like. Exemplary detergent additive formulations include but are not limited to granulates, in particular non-dusting granulates, liquids, in particular stabilized liquids or slurries.

Non-dusting granulates may be produced, *e.g*., as disclosed in U.S. Patent Nos. 4,106,991 and 4,661,452 and may optionally be coated by methods known in the art. Examples of waxy coating materials are poly(ethylene oxide) products (*e.g*., polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in, for example, GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition may be in any convenient form, *e.g*., a bar, a tablet, a powder, a granule, a paste, or a liquid. A liquid detergent may be aqueous, typically containing up to about 70% water, and 0% to about 30% organic solvent. Compact detergent gels containing about 30% or less water are also contemplated. The detergent composition can optionally comprise one or more surfactants, which may be non-ionic, including semi-polar and/or anionic and/or cationic and/or zwitterionic. The surfactants can be present in a wide range, from about 0.1% to about 60% by weight.

When included therein the detergent will typically contain from about 1% to about 40% of an anionic surfactant, such as linear alkylbenzenesulfonate, α-olefinsulfonate, alkyl sulfate (fatty alcohol sulfate), alcohol ethoxysulfale, secondary alkanesulfonate, α-sulfo fatty acid methyl ester, alkyl- or alkenylsuccinic acid, or soap.

When included therein, the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl-N-alkyl derivatives of glucosamine ("glucamides").

The detergent may contain 0% to about 65% of a detergent builder or complexing agent such as zeolite, diphosphate, triphosphate, phosphonate, carbonate, citrate, nitrilotriacetic acid, ethylenediaminetetraacetic acid (EDTA), diethylenetriaminepentaacetic acid, alkyl- or alkenylsuccinic acid, soluble silicates or layered silicates (*e.g*.,SKS-6 from Hoechst).

The detergent may comprise one or more polymers. Exemplary polymers include carboxymethylcellulose (CMC), poly(vinylpyrrolidone) (PVP), poly(ethylene glycol) (PEG), poly(vinyl alcohol) (PVA), poly(vinylpyridine-N-oxide), poly(vinylimidazole), polycarboxylates *e.g*., polyacrylates, maleic/acrylic acid copolymers), and lauryl methacrylate/acrylic acid copolymers.

The enzyme(s) of the detergent composition may be stabilized using conventional stabilizing agents, *e.g.,* as polyol (*e.g*., propylene glycol or glycerol), a sugar or sugar alcohol, lactic acid, boric acid, or a boric acid derivative (*e.g*., an aromatic borate ester), or a phenyl boronic acid derivative (*e.g*., 4-formylphenyl boronic acid). The composition may be formulated as described in WO 92/19709 and WO 92/19708.

It is at present contemplated that in the detergent compositions, in particular the enzyme variants, may be added in an amount corresponding to about 0.01 to about 100 mg of enzyme protein per liter of wash liquor (*e.g*., about 0.05 to about 5.0 mg of enzyme protein per liter of wash liquor or 0.1 to about 1.0 mg of enzyme protein per liter of wash liquor).

### 4.2 Cleaning Compositions

In the detergent applications, an AmyDSM90-related polypeptide is usually used in a liquid composition containing propylene glycol. The enzyme is solubilized in for example in propylene glycol by mixing in a 25% volume/volume propylene glycol solution containing 10% calcium chloride.

An AmyDSM90-related polypeptide thereof discussed herein can be formulated in detergent compositions for use in cleaning dishes or other cleaning compositions. These can be powders, gels, or liquids. The compositions can comprise the enzyme alone, or with other amylolytic enzymes and/or with other cleaning enzymes or bleach activating enzymes, and other components common to cleaning compositions.

Thus, a dishwashing detergent composition can comprise a surfactant. The surfactant may be anionic, non-ionic, cationic, amphoteric or a mixture of these types. The detergent can contain 0% to about 90% by weight of a non-ionic surfactant, such as low- to non-foaming ethoxylated propoxylated straight-chain alcohols.

The detergent composition may contain detergent builder salts of inorganic and/or organic types. The detergent builders may be subdivided into phosphorus-containing and non-phosphorus-containing types. The detergent composition usually contains about 1 % to about 90% of detergent builders. Examples of phosphorus-containing inorganic alkaline detergent builders, when present, include the water-soluble salts, especially alkali metal pyrophosphates, orthophosphates, and polyphosphates. An example of phosphorus-containing organic alkaline detergent builder, when present, includes the water-soluble salts of phosphonates. Examples of non-phosphorus-containing inorganic builders, when present, include water-soluble alkali metal carbonates, borates, and silicates, as well as the various types of water-insoluble crystalline or amorphous alumino silicates, of which zeolites are the best-known representatives.

Examples of suitable organic builders include the alkali metal; ammonium and substituted ammonium; citrates; succinates; malonates; fatty acid sulphonates; carboxymethoxy succinates; ammonium polyacetates; carboxylates; polycarboxylates; aminopolycarboxylates; polyacetyl carboxylates; and polyhydroxysulponates.

Other suitable organic builders include the higher molecular weight polymers and copolymers known to have builder properties, for example appropriate polyacrylic acid, polymaleic and polyacrylic/polymaleic acid copolymers, and their salts.

The cleaning composition may contain bleaching agents of the chlorine/bromine-type or the oxygen-type. Examples of inorganic chlorine/bromine-type bleaches are lithium, sodium or calcium hypochlorite, and hypobromite, as well as chlorinated trisodium phosphate. Examples of organic chlorine/bromine-type bleaches are heterocyclic N-bromo-and N-chloro-imides such as trichloroisocyanuric, tribromoisocyanuric, dibromoisocyanuric, and dichloroisocyanuric acids, and salts thereof with water-solubilizing cations such as potassium and sodium. Hlydantoin compounds are also suitable.

The cleaning composition may contain oxygen bleaches, for example in the form of an inorganic persalt, optionally with a bleach precursor or as a peroxy acid compound. Typical examples of suitable peroxy bleach compounds are alkali metal perborates, both tetrahydrates and monohydrates, alkali metal percarbonates, persilicates, and perphosphates. Exemplary activator materials are TAED, and glycerol triacetate. Enzymatic bleach activation systems may also be present in the formulation, *e.g*., such as perborate or percarbonate, glycerol triacetate and perhydrolase (*see, e.g.,* WO 2005/056783).

The cleaning composition may be stabilized using conventional stabilizing agents for the enzyme(s), *e.g.,* a polyol such as, *e.g.,* propylene glycol, a sugar or a sugar alcohol, lactic acid, boric acid, or a boric acid derivative (*e.g*., an aromatic borate ester).

The cleaning composition may also contain other conventional detergent ingredients, *e.g*., deflocculant material, filler material, foam depressors, anti-corrosion agents, soil-suspending agents, sequestering agents, anti-soil redeposition agents, dehydrating agents, dyes, bactericides, fluorescers, thickeners, and perfumes.

Although the present compositions and methods have been described with reference to the details below, it would be understood that various modifications can be made.

### 4.3 Methods of Assessing Amylase Activity in Detergent Compositions

Numerous α-amylase cleaning assays exist. Exemplary description of testing cleaning includes the following.

A "swatch" is a piece of material such as a fabric that has a stain applied thereto. The material can be, for example, fabrics made of cotton, polyester or mixtures of natural and synthetic fibers. The swatch can further be paper, such as filter paper or nitrocellulose, or a piece of a hard material such as ceramic, metal, or glass. For amylases, the stain is starch based, but can include blood, milk, ink, grass, tea, wine, spinach, gravy, chocolate, egg, cheese, clay, pigment, oil, or mixtures of these compounds.

A "smaller swatch" is a section of the swatch that has been cut with a single hole punch device, or has been cut with a custom manufactured 96-hole punch device, where the pattern of the multi-hole punch is matched to standard 96-well microtiter plates, or the section has been otherwise removed from the swatch. The swatch can be of textile, paper, metal, or other suitable material. The smaller swatch can have the stain affixed either before or after it is placed into the well of a 24-, 48- or 96-well microtiter plate. The "smaller swatch" can also be made by applying a stain to a small piece of material. For example, the smaller swatch can be a stained piece of fabric 5/8" or 0.25" in diameter. The custom manufactured punch is designed in such a manner that it delivers 96 swatches simultaneously to all wells of a 96-well plate. The device allows delivery of more than one swatch per well by simply loading the same 96-well plate multiple times. Multi-hole punch devices can be conceived of to deliver simultaneously swatches to any format plate, including but not limited to 24-well, 48-well, and 96-well plates. In another conceivable method, the soiled test platform can be a bead made of either metal, plastic, glass, ceramic, or other suitable material that is coated with the soil substrate. The one or more coated beads are then placed into wells of 96-, 48-, or 24- well plates or larger formats, containing suitable buffer and enzyme. In this case, supernatant can be examined for released soil either by direct absorbance measurement or after a secondary color development reaction. Analysis of the released soil might also be taken by mass spectral analysis. A further microscreening assay can be to deliver and secure a swatch, for example an indigo dyed denim, to a well of a multi-well plate, and add particles such as sand or larger particles such as for example garnet sieved to include particle 6 to 8, or 9 gauge, and agitate the plate so as to cause abrasion of the swatch by the added particles. This assay has found use in the assessment of cellulases in stone washing applications. The effectiveness of the enzyme can be judged by either color release (*e.g*., released indigo is dissolved in dimethylsulfoxide and absorbance at A₆₀₀ nm is measured) to the reaction buffer or by reflectance measurements of the abraded swatch.

When, for example, untreated BMI (blood/milk/ink) swatches are washed in detergent without bleach, a large portion of the ink is released even without the help of a protease. Adding a protease leads to a small increase in ink release, which can be hard to quantify over the large background. The present compositions and methods provide a treatment protocol that allows one to control the degree of fixation of a stain. As a result, it is possible to produce swatches that, for example, release varying amounts of stain when washed in the absence of the enzyme being tested. The use of fixed swatches leads to a dramatic improvement of the signal-to-noise ratio in the wash assays. Furthermore, by varying the degree of fixation, one can generate stains that give optimum results under the various cleaning conditions.

Swatches having stains of known "strength" on various types of material are commercially available (EMPA, St. Gallen, Switzerland; wfk--Testgewebe GmbH, Krefeld Germany; or Center for Test Materials, Vlaardingen, The Netherlands) and/or can be made by the practitioner (Morris and Prato, Textile Research Journal 52(4): 280 286 (1982)). Other test swatches include but are not limited to blood/milk/ink (BMI) stain(s) on a cotton-containing fabric, a spinach stain on a cotton-containing fabric, or grass on a cotton-containing fabric, and chocolate/milk/soot on a cotton-containing fabric.

A BMI stain can be fixed to cotton with 0.0003% to 0.3% hydrogen peroxide. Other combinations include grass or spinach fixed with 0.001% to 1% glutaraldehyde, gelatin and Coomassie stain fixed with 0.001% to 1% glutaraldehyde, or chocolate, milk and soot fixed with 0.001% to 1% glutaraldehyde.

The swatch can also be agitated during incubation with the enzyme and/or detergent formulation. Wash performance data is dependent on the orientation of the swatches in the wells (horizontal versus vertical), particularly in the 96-well plate. This would indicate that mixing was insufficient during the incubation period. Although there are a number of ways to ensure sufficient agitation during incubation, a plate holder in which the microtiter plate is sandwiched between two plates of aluminum can be constructed. This can be as simple as placing, for example, an adhesive plate sealer over the wells then clamping the two aluminum plates to the 96-well plate with any type of appropriate, commercially available clamps. It can then be mounted in a commercial incubator shaker. Setting the shaker to about 400 rpm results in very efficient mixing, while leakage or cross-contamination is efficiently prevented by the holder.

Trinitrobenzenesulfonic acid (TNBS) can be used to quantify the concentration of amino groups in the wash liquor. This can serve as a measure of the amount of protein that was removed from the swatch (*see e.g*., Cayot and Tainturier, Axial. Biochem. 249: 184-200 (1997)). However, if a detergent or an enzyme sample leads to the formation of unusually small peptide fragments (for example, from the presence of peptidases in the sample), then one will obtain a larger TNBS signal, *i.e*., more "noise".

Another means of measuring wash performance of blood/milk/ink or other stain is based on ink release. Proteolysis of protein on the swatches leads to the release of ink particles which can be quantified by measuring the absorbance of the wash liquor. The absorbance can be measured at any wavelength between 350 and 800 nm. The absorbance is measured at 410 nm or 620 nm. The wash liquor can also be examined to determine the wash performance on stains containing grass, spinach, gelatin or Coomassie stain. Exemplary wavelengths for these stains include 670 nm for spinach or grass and 620 nm for gelatin or Coomassie. For example, an aliquot of the wash liquor (typically 100-150 µL from a 96-well microplate, for example) is removed and placed in a cuvette or multiwell microplale. This is then placed in a spectrophotometer and the absorbance is read at an appropriate wavelength.

The system can also be used to determine an enhanced enzyme and/or detergent composition for dish washing, for example, using a blood/milk/ink stain on a suitable substrate such as cloth, plastic or ceramic.

In one aspect, the BMI stain is fixed to cotton by applying 0.3% hydrogen peroxide to the BMI/cotton swatch for 30 minutes at 25°C or by applying 0.03% hydrogen peroxide to the BMI/cotton swatch for 30 minutes at 60°C. Smaller swatches of approximately 0.25" are cut from the BMI/cotton swatch and placed in the wells of a 96-well microtiter plate. Into each well, a known mixture of a detergent composition and an enzyme such as a variant protein is placed. After placing an adhesive plate sealer onto the top of the microtiter plate, the microtiter plate is clamped to an aluminum plate and agitated on an orbital shaker at approximately 250 rpm for about 10 to 60 minutes. At the end of this time, the supernatants are transferred to wells in a new microtiter plate and the absorbance of the ink at 620 nm is measured. This can be similarly tested with spinach stains or grass stains fixed to cotton by applying 0.01% glutaraldehyde to the spinach/cotton swatch or grass/cotton swatch for 30 minutes at 25°C. The same can be done with chocolate, milk, and/or soot stains.

The present document is organized into a number of sections for ease of reading; however, the reader will appreciate that statements made in one section may apply to other sections. In this manner, the headings used for different sections of the disclosure should not be construed as limiting.

In order to further illustrate the compositions and methods, and advantages thereof, the following specific examples are given with the understanding that they are illustrative rather than limiting.

### EXAMPLES

### Example 1

### Cloning and isolation of the amyDSM90 gene

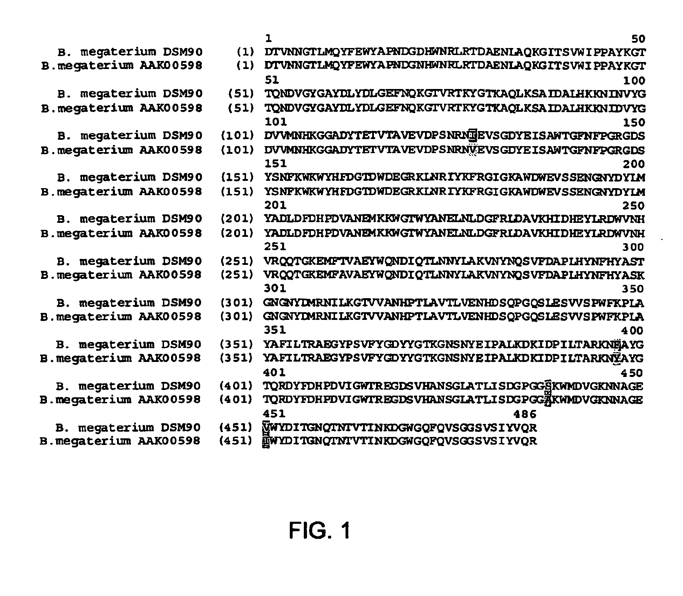

*Bacillus megaterium* strain DSM90 was obtained from Deutsche Sammlung von Mikroorganismen und Zelkulturen GmbH (DSMZ), Inhoffenstrasse 7B, 38124 Braunschweig, Germany. DNA was prepared and used for isolation of the *amyDSM90* α-amylase gene using a PCR primer set (SEQ ID NOs: 11 and 12) designed on the basis of the *Bacillus megaterium* AAK00598 amylase sequence (SEQ ID NO: 3). Translation of the sequenced nucleotide of clone pME602 indicated that the amylase from *B. megaterium* DSM90 (SEQ ID NO: 1) differed from the amylase of *B. megaterium* AAK00598 by eight amino acids (identity = 98.4%; FIG. 1).

Since this result was unexpected, the identity of *B. megaterium* strain DSM90 was verified by sequencing almost the entire 16S rRNA gene. Sequence results from automated base calling were confirmed manually to yield a final 1486 nts sequence for analysis (SEQ ID NO: 8). A BLAST search performed using SEQ ID NO: 8 as a query returned a large number of "hits" (with identities of 99-100%) to *B. megaterium* strains, although an rRNA sequence for *B. megaterium* strain DSM90 does not appear to be available in a public database. Based on the available evidence, it was concluded that the organism from which the subject DNA was obtained was a *B. megaterium* strain. For the purposes of the present disclosure, this strain will be referred to as DSM90, although strain taxonomy is not critical to the present compositions and methods.

A search for similar amylase sequences in public sequence databases was made using the BLASTp tool. Surprisingly, the search revealed that the most similar amylases (>94% identity) were derived from *B. anthracis* AAT3259 (SEQ ID NO: 4), *B. thuringiensis* AAT60457 (SEQ ID NO: 5), *B. cereus* AAP10417 (SEQ ID NO: 6), and not from other strains of *B. megaterium,* with the single exception of the amylase from *B. megaterium* AAK00598 (SEQ ID NO: 3). An alignment of these orthologous amylase sequences is shown in FIG. 2 and their relatedness (in terms of percent identity) is shown in (Table 1).

**Table 1. Percentage Identity of Orthologous Amylase Sequences**

| | *B. megaterium* DSM90 | *B. anthracis* AAT32659 | *B. thuringiensis* AAT60457 | *B. cereus* AAP10417 | *B. megaterium* AAK00598 |
|---|---|---|---|---|---|
| *B. megaterium* DSM90 | 100 | 95.1 | 94.4 | 98.6 | 98.4 |
| *B. anthracis* AAT32659 | | 100 | 99.0 | 95.3 | 95.1 |
| *B. thuringiensis* AAT60457 | | | 100 | 94.7 | 94.4 |
| *B. cereus* AAP10417 | | | | 100 | 98.6 |
| *B. megaterium* AAK00598 | | | | | 100 |

Phylogentically, *B. cereus, B. anthracis,* and *B. thuringiensis* form a tight cluster of highly related species and strains, only distantly related to *B. megaterium* (Ash et al., Letters in Applied Microbiology 13:202-206, 1991). Thus, while it may be expected on the basis of phylogenetic relatedness that *B. cereus, B. anthracis,* and *B. thuringiensis* contain similar amylases, it is surprising that they are highly similar to *B. megaterium* amylase. This suggests that AmyDSM90 is a member of an identifiable sub-group of amylases that also include those from *B. cereus, B. anthracis,* and *B. thuringiensis.* It is noteworthy that database annotations describe these *B. cereus, B. anthracis,* and *B. thuringiensis* amylases as "cytoplasmic", *i.e*., not exported or secreted outside the cell, which observations are contradicted by the present findings.

### Example 2

### Expression of AmyDSM90 in Bacillus lichenformis

To *express B. megaterium* DSM90 amylase (AmyDSM90) in *Bacillus lichenformis,* intermediate constructs were made by using the pHPLT vector. pHPLT (FIG. 3; Solingen et al., Extremophiles 5:333-41, 2001) contains the thermostable amylase LAT promoter (pLAT) and the LAT signal peptide (pre LAT) of *B. licheniformis* (SEQ ID NO: 7), followed by *Pst*I and *Hpa*I restriction sites for cloning.

Genomic *DNA* of *B. megaterium* DSM90 was prepared from 1 nil cell pellet of an overnight grown culture (Tryptic Soy Broth at 30°C) using the MASTERPURE Gram Positive DNA purification Kit from Epicentre according to the manufacturer's protocol. The *amyDSM90* gene was amplified by PCR on a thermocycler with PURETAQ READY-TO-GO PCR Beads (GE Healthcare) according to the instructions of the manufacturer (annealing temperature of 55°C.) using the aforementioned primers based on the *B. megaterium* AAK00598 amylase (SEQ ID NOs: 11 and 12). The resulting PCR fragment was digested with restriction enzymes *Pst*I and *Hpa*I, and ligated with T4 DNA ligase into digested pHPLT pDNA (50 ng/µl, digested with *Pst*I and *Hpa*I restriction enzymes) according to the instructions of the supplier (Roche Applied Science, Indianapolis, IN, USA). The ligation mixture was transformed into *B. subtilis* strain WW120 (also known as BG3934; *amyE*::*xylRPxylAcomK-ermC*). The transformation reaction was plated on LB agar plates containing 10 ppm neomycin and 1% w/v insoluble starch. About 50-100 colonies were obtained.

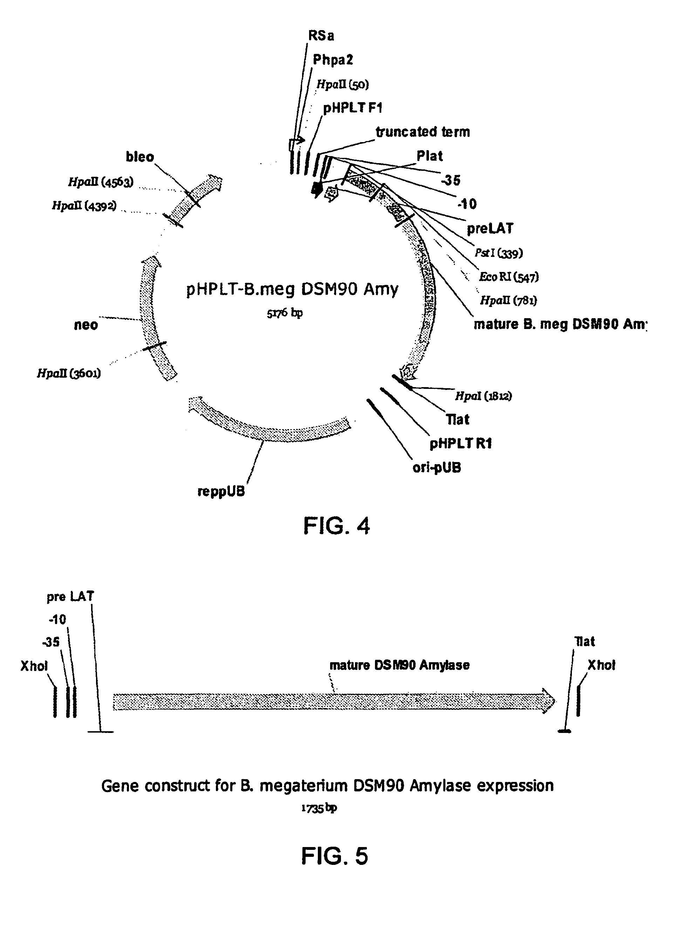

Twenty colonies were evaluated for the presence of insert using colony PCR and sequencing. For the colony PCR, each *B. subtilis* transformant was suspended in 20 µL of sterile water of which 2 µL was used in a PCR reaction containing a READY-TO-GO TAQ PCR bead (Amersham), 22 µL sterile water, 0.5 µL 25 µM pHPLT-F1 primer (SEQ ID NO: 13) and 0.5 µL 25 µM pHPLT-R1 primer (SEQ ID NO: 14). The cycling conditions were 94°C for 4 minutes once, and 94°C for 1 minute, 53°C for 1 minute, and 72°C for 2 minutes for a total of 30 cycles, followed by 7 minutes at 72°C for one final cycle. 5 µL, of the PCR products were digested with Exo-SapIT (Amersham) to remove dNTPs and primers before sequencing. Sequencing was performed using primers pHPLT-seq-F1 (SEQ ID NO: 15) and pHPLT-seq-R1 (SEQ ID NO: 16) to verify the sequence of the insert. The resulting plasmid was designated pME602.13 (also referred to as pHPLT-B.meg DSM90 Amy, as on the plasmid map shown in FIG. 4). The cloned amylase gene had eight amino acid changes compared to the AAK00598 amylase (FIG. 1). This plasmid can be used directly for expression of AmyDSM90-related polypeptides, as described in Example 3.

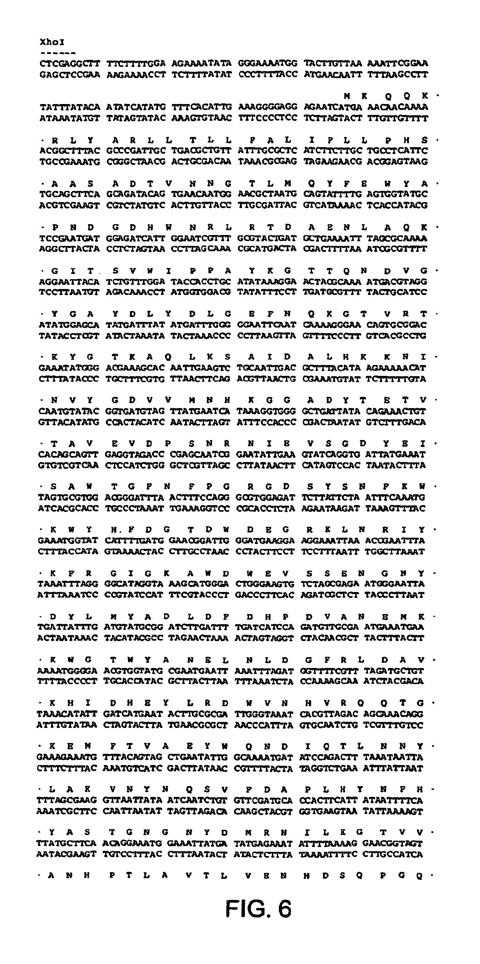

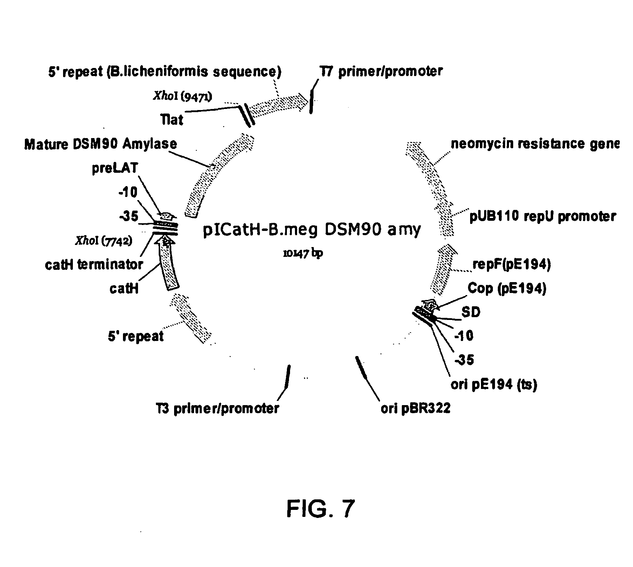

The pHPLT-B.meg DSM90 Amy plasmid (DSM90-wild-type) was then used to produce *B. lichenformis* AmyDSM90 expression strains, in which AmyDSM90 was expressed in as a fusion protein with the signal peptide of *B. lichenformis* α-amylase (LAT) (FIGS. 5 and 6, referring to SEQ ID NOs: 9 and 10). PCR amplification was performed using the primers AmyPlatFW (SEQ ID NO: 17) and AmyTlat_RV (SEQ ID NO: 18) and plasmid pHPLT-B.meg DSM90 Amy as a template to produce an *Xho*I fragment that contained the LAT-DSM90 Amy precursor (*i.e*., immature with signal sequence) gene flanked by the complete LAT promoter at the 5' end, and the LAT terminator at the 3' end. PCR was performed on a thermocycler with PHUSION High Fidelity DNA polymerase (Finnzymes OY, Espoo, Finland) according to the manufacturer's instructions (annealing temperature of 55°C). The resulting PCR fragment was digested with restriction enzyme *Xho*I and ligated with T4 DNA ligase into *Xho*I-digested pICatH according to the supplier's instructions (Invitrogen, Carlsbad, Calif. USA). The ligation mixture was transformed into *B. subtilis* strain SC6.1 as described in U.S. Patent Application US20020182734 (International Publication WO 02/14490). The sequence of the *Xho*I insert containing the LAT-AmyDSM90 precursor gene was confirmed by DNA sequencing (BaseClear, Leiden, The Netherlands) and the correct plasmid clones was designated pICatH-B.meg DSM90 amy(ori1) (FIG. 7).

The pICatH-B.meg DSM90 amy (ori1) plasmid was then transformed into *B. licheniformis* strains BML612 and BML780 (derivatives of BRA7 and BML612, respectively; WO2005111203) at the permissive temperature (37°C). From each transformation, one neomycin resistant (neoR) and chloramphenicol resistant (CmR) transformant was selected (designated BML612 (plCatH-AmyDSM90(ori1) and BML780(pICatH-AmyDSM90 (ori1), respectively). These plasmids were integrated into the catH region on the *B. licheniformis* genome by growing each transformer strain at the non-permissive temperature (50°C) in a medium containing 5 µg/ml chloramphenicol. One chloramphenicol resistant clone of each strain was selected and grown again at the permissive temperature with chloramphenicol, but without neomycin, for several generations to loop-out vector sequences, and then one neomycin sensitive (neoS), CmR clone of each strain was selected for further analysis. In these clones, vector sequences of plCatH on the chromosome are excised (including the neomycin resistance gene) and only the catH-LATAmyDSM90 cassette remains.

The catH-LATAmyDSM90 cassette on the chromosome was amplified by growing each strain in/on media with increasing concentrations of chloramphenicol. After several rounds of amplification, one clone of each strain that was resistant to 75 µg/ml chloramphenicol was selected and designated BML612-DSM90amyCAP75 and BML780-DSM90amyCAP75, respectively. To verify AmyDSM90 expression, the strains were grown for 48h at 37°C on a Heart Infusion (Bacto) agar plate with 1% Starch Azure (Potato starch covalently linked with Remazol Brilliant Blue R; Sigma-Aldrich, S7629). A clearing zone, indicative of amylolytic activity, was clearly visible around the colonies, suggesting that a substantial amount of enzymatically active AmyDSM90 was expressed in the *B. licheniformis* strains.

### EXAMPLE 3

### Expression of AmyDSM90 in Bacillus subtilis

*B. subtilis* strain WW 120 transformed with plasmid pME602.13 (described in Example 2) secrete enzymatically active AmyDSM90-related polypeptides as demonstrated by halo formation on starch plates following iodine staining. To express AmyDSM90-related polypeptides, cultures of this strain were typically grown at 37°C for 60 to 72 hours with agitation at 250 rpm in the following medium (per liter): 10 g Soytone, 75 g glucose, 7.2 g urea, 40 mM MOPS, 4 mM Tricine, 3 mM dibasic potassium phosphate, 21.4 mM KOH, 50 mM NaCl, 276 µM potassium sulfate, 528 µM magnesium chloride, 50 µM trisodium citrate dihydrate, 100 µM calcium chloride dihydrate, 14 µM ferrous sulfate heptahydrate, 5.9 µM manganese sulfate dihydrate, 5.7 µM zinc sulfate monohydrate, 2.9 µM cupric chloride dihydrate, 4.2 µM cobalt hexahydrate, and 4.5 µM sodium molybdate dihydrate. For a 1L volume, all components except for Soytone were mixed in 500 mL, sterile filtered, and added to an equal part of 2X Soytone, which had been sterilized by autoclaving. Trace metals and citrate were be made up as a 100X or 1000X stock solutions. Buffers, potassium hydroxide, sodium chloride, potassium sulfate, and magnesium chloride and trace metals were be made up as a 10X stock solutions. After all components were mixed, the pH was adjusted to 7.3. Prior to use this medium was supplemented with 20 mM calcium chloride and 10 mg/L Neomycin-trisulfate. All experiments described herein use AmyDSM90 expressed in *B. subtilis.*

### EXAMPLE 4

### Purification of AmyDSM90

Growth media from the shake flask (500 mL) described in Example 3, which included expressed AmyDSM90, was concentrated to a 25mL volume. 25ml of 50 mM MES buffer, pH 5.8, containing 2 mM calcium chloride was then added to the concentrate, followed by 6.6 g ammonium sulfate to give a final ammonium sulfate concentration of 1 M. After filtering, this sample was applied to a 5 mL phenyl sepharose column equilibrated with a buffer containing 50 mM MES, pH 5.8, 2 mM calcium chloride, and 1M ammonium sulfate. After loading, the column was washed with eight volumes the same buffer as used for equilibration. AmyDMS90-related polypeptide was eluted from the column with a gradient beginning with the equilibration/wash buffer and ending with 50 mM MES, pH 5.8, 2 mM calcium chloride buffer (no ammonium sulfate) over twelve column volumes. The latter conditions were continued for seven column volumes, and then 40% propylene glycol was included in the buffer for five column volumes. The column was washed with water in a reverse flow direction for five column volumes and in a forward direction for ten column volumes. The amylase began eluting nine column volumes into the gradient and continued eluting over two column volumes.

Alpha-amylase activity was measured using the Megazyme amylase activity assay, and all active fractions were pooled, concentrated (using 5K VIVASPIN 20 mL centrifugal concentrators), and buffer-exchanged three times into 50 mM MES, pH 5.8, 2 mM calcium chloride buffer to remove residual ammonium sulfate.

### EXAMPLE 5

### Expression of AmyDSM90-related polypeptides in Bacillus licheniformis

Variants of AmyDSM90 [*e.g*., including (a) R179-G180 deletions (ΔRG), (b) an M200L substitution, and (c) a combination of these mutations] were generated using the QUICK CHANGE multi site mutagenesis method (Stratagene, La Jolla, CA).

The individual variants (*i.e*., separately including the R179 and G180 deletion and the M200L substitution) were first created using the QUIK-CHANGE multi site mutagenesis kit along with respective primer pairs RG F (SEQ ID NO: 21)/RG R (SEQ ID NO: 22), and M200F (SEQ ID NO: 19)/M200R(SEQ ID NO: 20). The ΔRG variant served as template for a second QUICK-CHANGE multisite reaction using primer pair M200F/M200R in order to combine both the RG deletion and the M200L substitution. All three variants were created using the protocol outlined below:

Prior to Quik-CHANGE mutagenesis, pHPLT-B.meg DSM90 Amy was methylated using dam methylase (NEB, Massachusetts, USA). pHPLT-B.meg DSM90 Amy served as template to make the first two variants using the QUIK-CHANGE multi site mutagenesis kit, following the kit protocol, with the exception of complementary primers being used in the reactions instead of single forward primers. 1 µL of the QUICK-CHANGE reaction served as template for rolling circle amplification using the GE Healthcare Illustra Templiphi kit (GE Healthcare) following the kit protocol. The rolling circle reaction was diluted ten fold, and 1 µL was transformed into 100 uL of competent *Bacillus subtilis* BG6006 cells (*degUHy32, oppA, dspoII3501, amyE::xylRPxylAcomK- ermC, daprE, duprE, depr, dispA, dbpr, dvpr, dwprA, dmpr-ybfJ, dnprB*) (US20050202535A1). The transformation was plated on LB agar plates containing 10 ppm neomycin and 1% insoluble starch. About 100 colonies were obtained. Four colonies were evaluated for the presence of the desired mutations using colony PCR and sequencing. For the colony PCR, each *B. subtilis* transformant was resuspended in 20 µL of sterile water of which 2 µL was used in a PCR reaction containing a READY-TO-GO TAQ PCR bead from Amersham, 22 µL of sterile water, 0.5 µL of 25 µM pHPLT-F1 primer (SEQ ID NO: 13) (5'-TACATATGAGTTATGCAGTTTG-3') and 0.5 µL of 25 µM pHPLT-R1 primer (SEQ ID NO: 14) (5'-GTTATGAGTTAGTTCAAATTCG-3'). The cycling conditions were 94°C for 4 minutes once; 94°C for 1 minute, 53°C for 1 minute, 72°C for 2 minutes, for a total of 30 cycles; followed by 7 minutes at 72°C for one final cycle. 5 µL of the PCR products were digested with Exo-SapIT from Amersham to remove dNTPs and primers before sequencing. Sequencing was carried out with primers pHPLT-seq-F1 (SEQ ID NO: 15) and pHPLT-seq-R1 (SEQ ID NO: 16) to verify the presence of the desired mutations.

The pHPLT-B.meg DSM90 Amy plasmids (*i.e*., having the R179-G180 deletion, M200L substitution, or both) served as templates to construct *Bacillus licheniformis* AmyDSM90 expression strains, as described in Example 2.

### EXAMPLE 6

### Expression of AmyDSM90-related Polypeptides Using Codon-Modified Genes

Synthetic, codon-optimized DNA fragments encoding each of four AmyDSM90-related polypeptides (*i.e*., naturally occurring orthologues) were produced by GeneArt GmbH (Regensburg, Germany) and used to construct additional *Bacillus subtilis* strains (Table 2).

**Table 2. Synthetic DNA fragments containing modified codons**

| DNA Fragment | Amylase Gene | SEQ ID NO |
|---|---|---|
| 0706267 | *B. anthracis* AAT32659 | 23 |
| 0706265 | *B. cereus* AAP10417 | 24 |
| 0706266 | *B. thuringiensis* AAT60457 | 25 |
| 0706264 | *B. megaterium* AAK00598 | 26 |

The pHPLT vector (FIG. 3; Example 2) was used for the expression of these AmyDSM90-related polypeptides. The synthetic genes (*i.e.,* SEQ ID NO 23, SEQ ID NO 24, SEQ ID NO 25, and SEQ ID NO 26) were digested with restriction enzymes *Pst*I and *Hpa*I*,* and ligated with T4 DNA ligase into pHPLT pDNA (50 ng/µl) digested with *Pst*I and *Hpa*I according to the instructions of the supplier (Roche Applied Science, Indianapolis, IN, USA). The ligation mixture was transformed into *B. subtilis* strain WW 120 and subjected to sequence analysis as described in Example 2.

To verify expression of the AmyDSM90-related polypeptides, each of the transformed bacteria were grown for 24h at 37°C on a Heart Infusion (Bacto) agar plates with 10 mg/l neomycin and 1% starch azure. A clearing zone, indicative of amylolytic activity, was clearly visible around all the colonies. These results show that substantial amounts of the AmyDSM90-related polypeptides were expressed in *B. subtilis* and had amylase activity.

### EXAMPLE 7

### Megazyme Assay for α-Amylase Activity

Megazyme assays to measure α-amylase activity were performed as follows:

### Materials:

25 mM Bis Tris Propane buffer pH 6.9 and 2 mM CaCl₂,
BPNPG7 substrate, 5.45 mg/mL (Amylase HR Reagent, *p*-nitrophenyl a-D-maltoheptaoside (blocked) Catalogue Number: R-AMHR4 Megazyme International),
amyloglucosidase, 10 U/mL, and
α-glucosidase, 10 U/mL.

### Method:

1. Substrate was prepared by adding 10 mL distilled water to a vial containing BPNPG7 with amyloglucosidase/α-glucosidase. 1 mL aliquots were prepared and stored at -20°C.
2. A thermomixer was set to 43°C.
3. A plate reader was adjusted to the following settings: 40°C; 405 nm, kinetic, 45 minutes, 15 second interval, pre-mix for 7 seconds.
4. Enzyme dilutions were prepared up to 1 ppm with BTP pH 6.9 and 2 mM CaCl₂ buffer, and stored on ice.
5. 10 µL of substrate were added to 150 uL of BTP pH 6.9 and 2 mM CaCl₂ buffer. The substrate solution was prepared in bulk and 160 µL of solution was distributed to each microtiter plate well of a plate prewarmed to 40°C for at least 10 minutes.
6. With the plate remaining on the warm platform, 10 µL of enzyme was added to each well.
7. Plates were immediately transferred to the plate reader, and the rate of the enzymatic reaction was monitored for 45 min or until sufficient linear data was obtained.
8. Specific activity was calculated by dividing the obtained rate (milliOD/min) by the µg enzyme used in assay.

### EXAMPLE 8

### Performance of AmyDSM90-related Polypeptides in a Screening Assay for Cleaning

Partially purified AmyDSM90-related polypeptides (Example 4) was analyzed in a 96-well CS28-orange-dyed-rice-starch-soiled-fabric-swatch micro-applications cleaning assay. Using a fabric punch, ¼-inch discs were cut from CS28 rice starch stained fabric (Test Fabrics Cat. No. CS-28; Test Fabrics Inc) and two discs placed in each well of flat-bottomed 96-well assay plates. Prior to performing the amylase assay, the swatches were pre-washed to remove any loosely bound dye by adding 200 µL water to each well, placing the assay plate in a thermomixer, and incubating at 25°C with shaking at 750 rpm for 1 hour. The wash-liquor was removed from each well and the swatch discs were rinsed once in 200 µL water. The rinse water was immediately discarded and the washed swatch discs were air dried in the assay plates at 37°C.

For the α-amylase cleaning assay, a preselected buffer was added to the wells and equilibrated to a preselected temperature. In the present experiment, the assay was performed in in 25 mM BTP (pH 8.0) or in 25 mM CAPS (pH 10.3), with each buffer solution additionally containing 2 mM CaCl₂. 200 µL of a preselected buffer was added to each well and allowed to equilibrate to the desired temperature (*i.e*., 20°C or 40°C) for 15 minutes. 10 µL of enzyme solution was added to each well and the plates incubated at the same temperature with shaking at 750 rpm for 1 hour. Enzyme performance was established by the amount of enzyme-dependent color release as quantified spectrophotometrically at 488 nm following the transfer of 150 µL assay solution to a fresh microtiter plate. For additional information on the assay, see U.S. Patent No. 7,122,334.

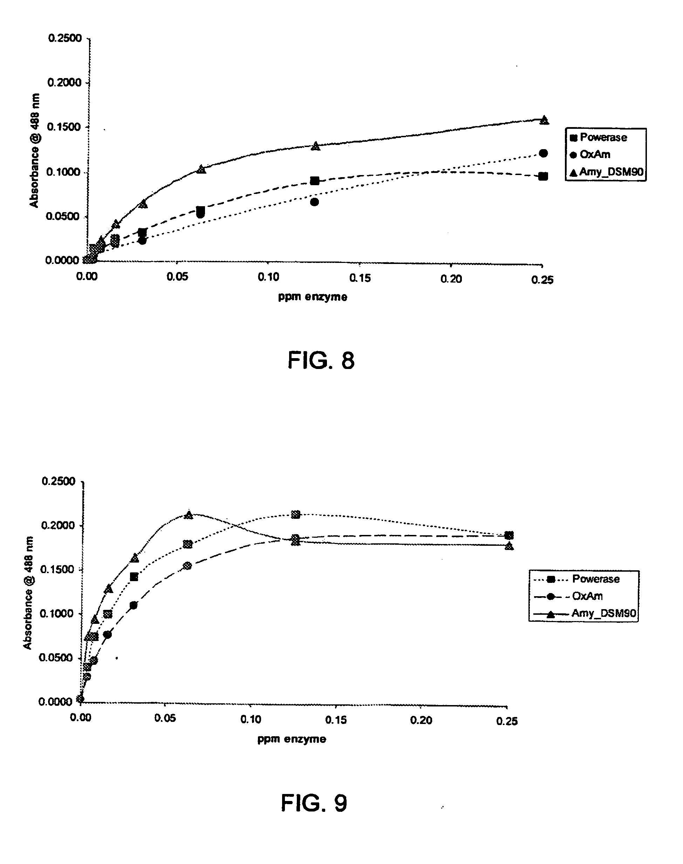

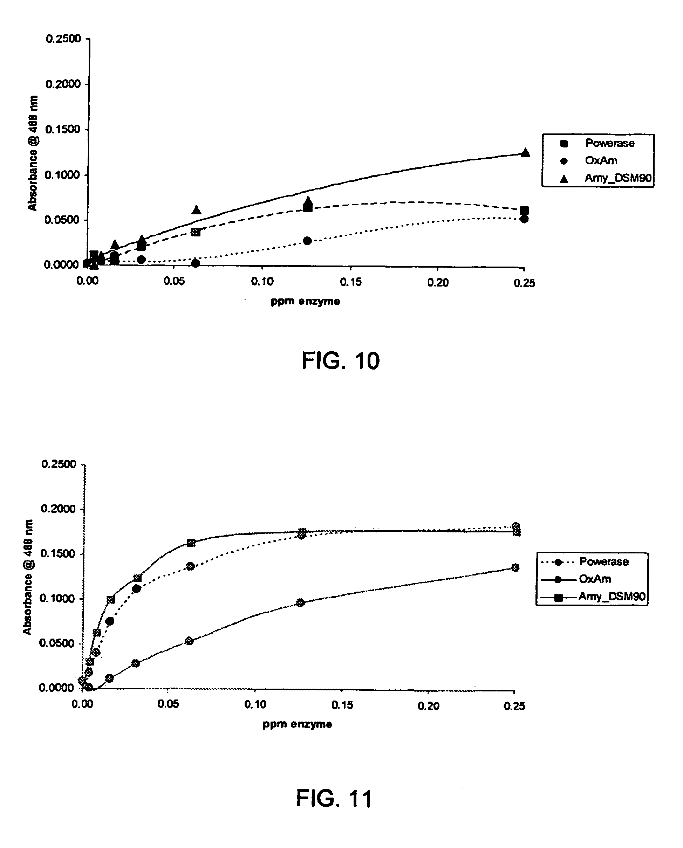

The results are shown in the graphs in FIGS. 8-11. AmyDSM90 was highly efficient at removing starchy stains from textile swatches at 40°C at both pH 8 and pH 10.3, where it performed better than the control enzyme (*i.e*., OXAM/PURASTAR®, Genencor International. Palo Alto, CA, USA). Surprisingly, the enzyme was also remarkably efficient at the lower temperature, i.e., 20°C, at both pH 8 and pH 10.3, where it showed better stain removal than the control enzymes OXAM/PURASTAR® and POWERASE® (both from Genencor International. Palo Alto, CA, USA).

The described 96-well version of this assay is suitable for high-throughput screening. A 24-well plate version of the assay, using larger swatches for which reflectance can be measured, was used to confirm the results. The two measurements (*i.e.*, supernatant absorbance and swatch reflectance) showed nearly perfect correlation (coefficient of determination, r², had a value of 0.99), thereby confirming the results.

### EXAMPLE 9

### Wash Performance of AmyDSM90-related Polypeptides in Detergent

Wash performance tests are conducted using CS28 rice starch stained fabric swatches with an indicator dye bound to the starch (Test Fabrics Cat. No. CS-28 Test Fabrics Inc.). Prior to assays, the dyed test fabric is prewashed with distilled water for 1 hour and air-dried. The prewash of the test fabric removes any poorly-bound indicator dye prior to the assay. Alternatively, the swatches are pre-washed after they have been loaded into the wells of a microtiter plate (as described in Example 8).

¼-inch disks are cut from the pre-washed fabric and placed into 96-well plates. 190 µL detergent solution is added into each well (*e.g*., IEC-A* Base Detergent (Cat. No. 88010-1; WFK Testgewebe GmbH, Germany). The plate with swatches and detergent is pre-incubated at 40°C for 15 minutes. The amylase assay is initiated with addition of 0-1 ppm purified AmyDSM90-related polypeptide from Example 4 or 0 to 1 ppm of a commercial α-amylases (e.g., OXAM/PURASTAR®) or POWERASE® (as controls) in a 10 µL volume. Wash performance is determined by measuring color release of the indicator dye into the solution phase measured by spectrophotometry at 488 nm.

### EXAMPLE 10

### Various AmyDSM90-related Polypeptides in a Screening Assay for Cleaning

Naturally occurring variants of AmyDSM90, namely *Bacillus anthracis* AAT32659 amylase, *Bacillus cereus* AAP10417 amylase, *Bacillus thuringiensis* AAT60457 and *Bacillus megaterium* AAK00598 amylase, were expressed in *Bacillus subtilis* BG6006 and tested for cleaning efficiency, essentially as described in Example 8. The assay was carried out in 25 mM HEPES (pH 8.0) or in 25 mM CAPS (pH 10.3) buffer, each buffer having a water hardness of 150 ppm Ca²⁺ and Mg²⁺ (2:1 Ca:Mg). 209 µL buffer solution was added to each well of a 96-well microtiter plate and allowed to equilibrate at the desired temperature (*i.e*., 40°C) for 15 minutes. 11 µL of purified enzyme solution was added to each well and the plates incubated at the same temperature with shaking at 750 rpm for 1 hour. As before, enzyme performance was based on the amount of enzyme dependent color released, which was quantified spectrophotometrically at 488 nm following the transfer of 150 µL reaction solution to a fresh microtiter plate.

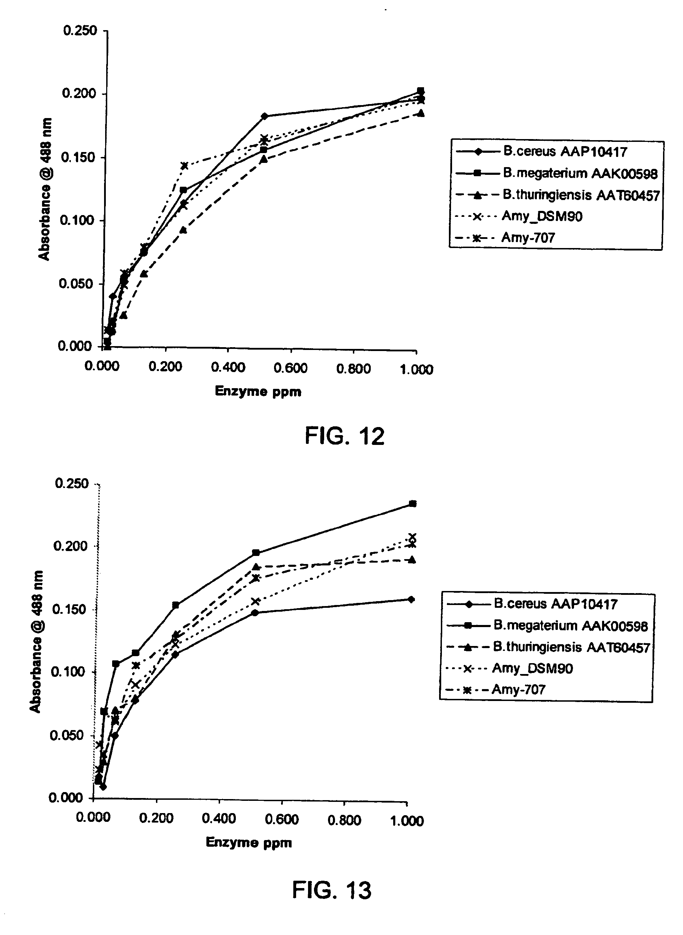

The results shown in FIGS. 12 and 13 demonstrate the similar cleaning ability (*i.e*., starch removal) of orthologous amylases compared to AmyDSM90 and Amy707 (Tsukamoto et al., Biochem. Biophys. Res. Comm. 151:25-31, 1988).

### EXAMPLE 11

### Terg-o-tometer testing of AmyDSM90-related Polypeptides

The Terg-o-tometer provides an intermediate laboratory-scale testing assay for detergents and enzymes, which simulates top-loading, paddle action washing machines common in North America. The conditions and procedures use to perform the assay are described, below:

### Swatches:

EMPA161 dyed maize starch on cotton (EMPA Testmaterialien AG, St. Gallen, Switzerland) and
CS-28 colored rice starch on cotton (Test fabrics, Inc., Center for Testmaterials, Vlaardingen, Netherlands).

### Detergents:

AATCC liquid, pH 7 (1.5 g/L) and

AATCC powder, pH 10.

### Amylases:

*B. megaterium* DSM90 (AmyDSM90),
*B. megaterium* AAK00598, and
OxAm (Purastar®) as a control.

### Conditions:

1 liter Terg-o-tometer,
4 starch soiled swatches plus 4 white bleached cotton swatches for ballast,
6 gpg (approx. 100 ppm [3:1, Ca:Mg]) water hardness, and
23°C wash temperature.

### Method:

The centers of soil swatches were pre-read two times on a black background using a Minolta reflectometer. 1L of deionized water was added to the terg-o-tometer, followed by a preselected detergent dosage and salts to produce the preselected water hardness. The solution was mixed for 5 minutes and allowed to reach a wash temperature of 74°F. The pH of the terg-o-tometer was measured and test amylases were added. Four starch-stained swatches and four white bleached cotton swatches were added and the terg-o-tometer was started. The swatches were mixed in the detergent solution at 100rpm for 15 minutes. Following incubation, the swatches were transferred to a 4L plastic beaker, rinsed under running tap water and running deionized water (from below) for 3 minutes, and placed in a front-loading washer for a spin cycle. The swatches were dried overnight by placing them flat on a clean sheet. The center of soil on the swatches was read two times on top of a black background. The results are recorded as soil removal index (% delta SRI).

### Results:

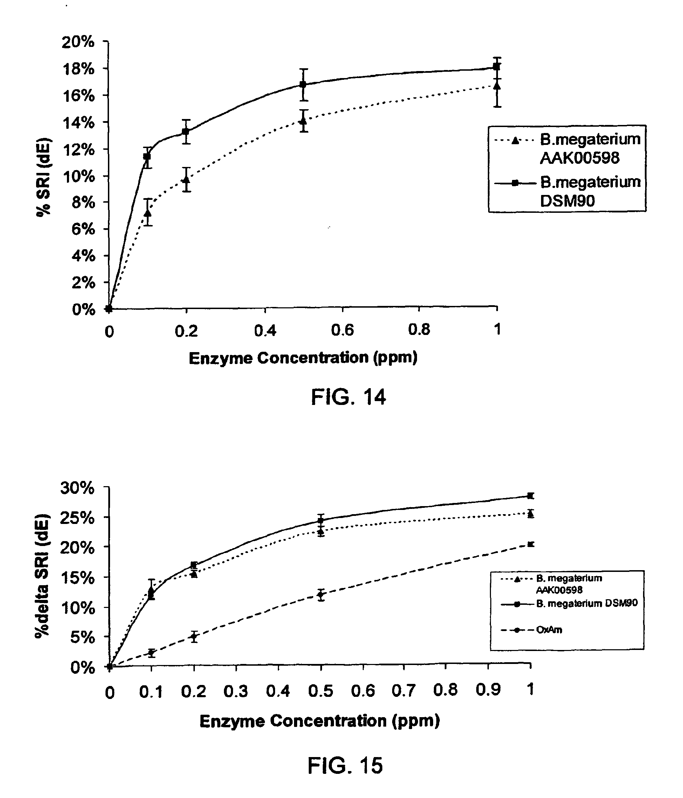

The results are shown in FIGS. 14 to 16 and demonstrate the superior laundry wash performance of the present amylases in liquid and powder detergents compared to OXAM (PURASTAR®).

### EXAMPLE 12

### Launder-o-meter testing of AmyDSM90-related Polypeptides

The Launder-o-meter (LOM) provides an intermediate, laboratory scale testing of detergents and enzymes which simulates front-loading, drum-type washing machines common in Europe. The conditions and procedures use to perform the assay are described, below:

### Swatches:

EMPA161 dyed maize starch on cotton (EMPA Testmaterialien AG, St. Gallen, Switzerland) and
CS-28 colored rice starch on cotton (Test fabrics, Inc., Center for Testmaterials, Vlaardingen, Netherlands).

### Detergents:

IEC A Standard Detergent, pH10.7 (6.16 g/L),
IEC A with Bleach, pH 10.4,
Commercial gel laundry detergent.

### Amylases:

*B. megaterium* DSM90 (AmyDSM90), wild-type,
*B. megaterirum* DSM90 mutant M200L,
*B. megaterium* DSM90 mutant ΔRG,
*B. megaterium* DSM90 mutant M200L and ΔRG,
OXAM (PURASTAR®), as a control, and
*B. licheniformis* amylase (LAT, Genencor International) as a control.

### Conditions:

4 starch soiled swatches per test,
12 gpg (approx. 200 ppm [3:1, Ca:Mg]), water hardness, and
40°C wash temperature.

### Method:

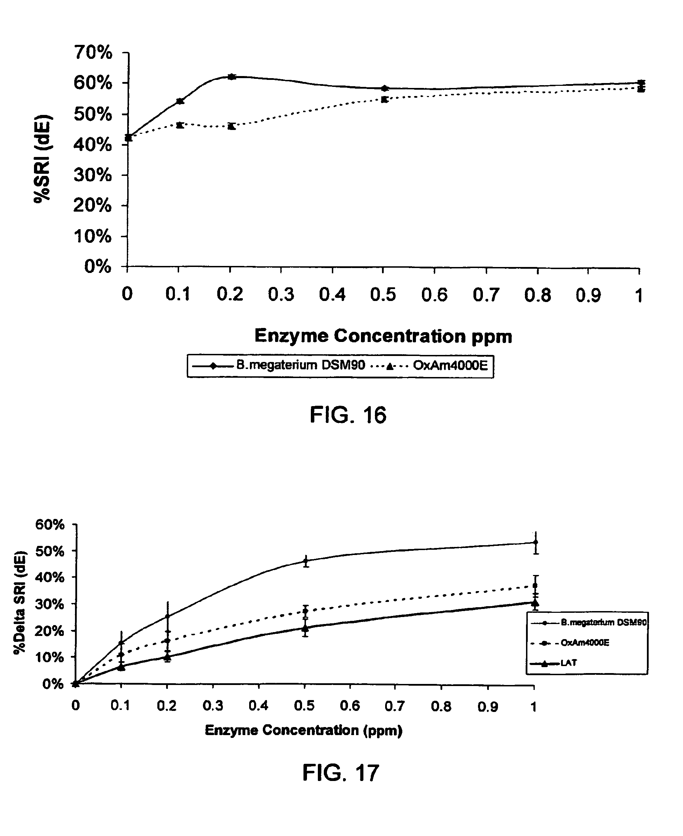

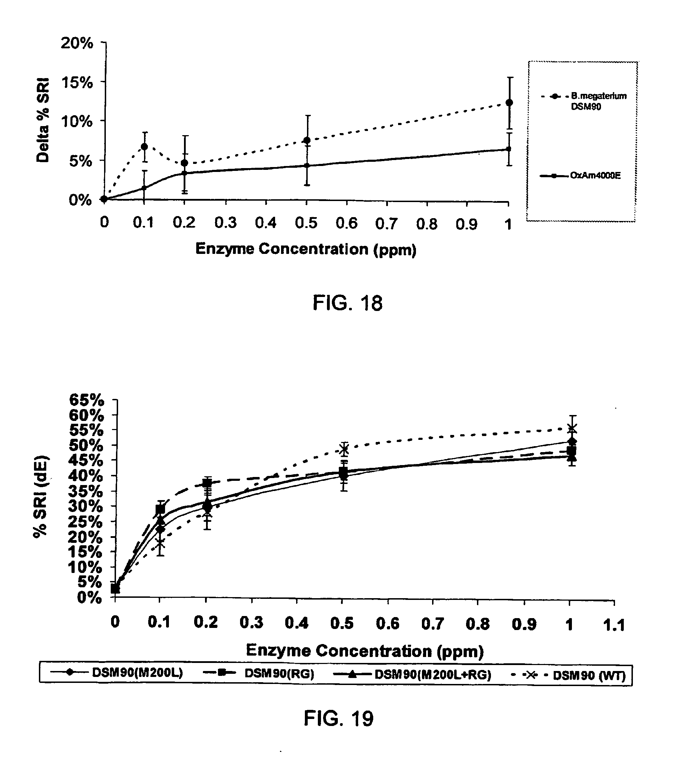

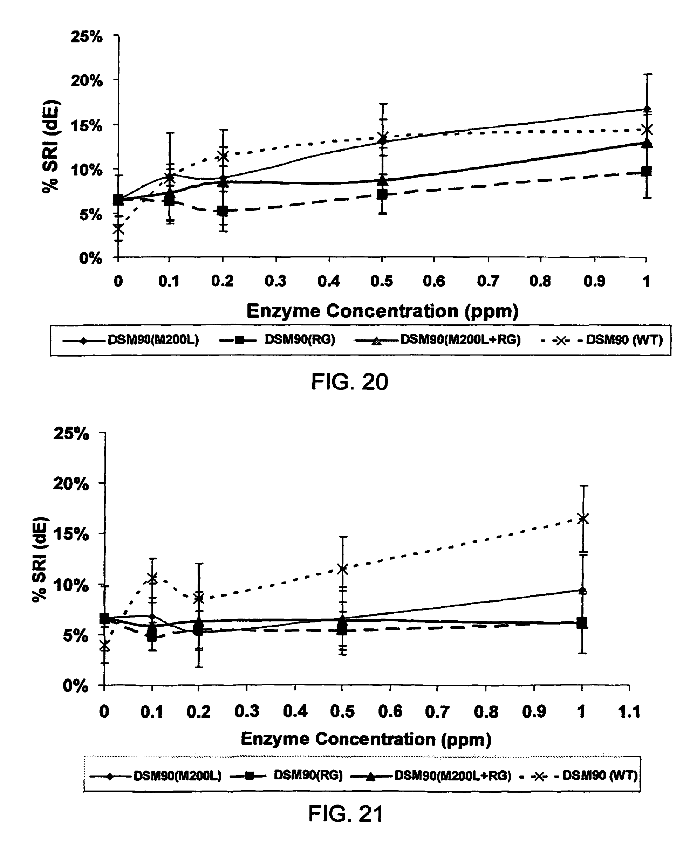

The LOM was set to the appropriate temperature. The wash cycle was started at room temperature and the LOM was allowed to ramp up to the desired temperature in the 45 min wash cycle. The center of soil swatches was pre-read two times on a black background using a 50 mm Minolta reflectometer. 200 mL of wash solution was added. Stainless steel balls (6) and test enzyme samples were added and mixed well, followed by the addition of the swatches. The test beakers were covered and secured with a clip and placed into the LOM. After 45 min incubation, the swatches were removed from the test beaker, excess water squeezed out, and transferred to a 4L plastic beaker. The swatches were rinsed under running tap water for 3 minutes, placed in a front-loading washer for a spin cycle, and air-dried flat overnight. The center of soil on the swatches was read two times on top of a black background. Results are recorded as soil removal index (% delta SRI). The results are shown in FIGS. 17 to 21. AmyDSM90 has an improved washing performance compared to the commercial enzymes (OXAM and LAT) in both standard detergent and commercially available detergent, for example the variant M200L gives a performance benefit in bleach containing detergent (FIGS. 17 and 18).

## Claims

1. An isolated α-amylase having at least 80% amino acid sequence identity to AmyDSM90 (SEQ ID NO: 1) and having:
a) an aspartic acid at a position corresponding to position 21 of AmyDSM90 (SEQ ID NO: 1);
b) an asparagine at a position corresponding to position 97 of AmyDSM90 (SEQ ID NO: 1); and
c) an isoleucine at a position corresponding to position 128 of AmyDSM90 (SEQ ID NO: 1).

2. The α-amylase of claim 1, having at least 90% identity to the amino acid sequence of SEQ ID NO: 1.

3. The α-amylase of claim 1, having at least 95% identity to the amino acid sequence of SEQ ID NO: 1.

4. The α-amylase of claim 1, having a deletion of the residues corresponding to R179 and G180 of AmyDSM90 (SEQ ID NO: 1).

5. The α-amylase of claim 1 or claim 4, having an amino acid other than methionine at a position corresponding to position 200 of AmyDSM90 (SEQ ID NO: 1).

6. The α-amylase of claim 1, having the amino acid sequence of SEQ ID NO: 1, SEQ ID NO: 28, SEQ ID NO: 29, or SEQ ID NO: 30.

7. A composition comprising the polypeptide of any one of claims 1-6.

8. The composition of claim 7, wherein the composition is a cleaning composition, e.g. wherein the composition is effective for removing starchy stains from laundry, dishes or a textile.

9. A method for removing a starchy stain from a surface, comprising
incubating the surface in the presence of a aqueous composition comprising an effective amount of an α-amylase according to any one of claims 1 to 6, and allowing the α-amylase to hydrolyse starch components present in the starchy stain to produce smaller starch-derived molecules that dissolve in the aqueous composition, thereby removing the starchy stain from the surface.

10. The method of claim 9, wherein the surface is a textile surface, a dish surface or a laundry surface.

11. An isolated polynucleotide encoding an α-amylase according to any one of claims I to 6.

12. An isolated polynucleotide according to claim 11 having the sequence of SEQ ID NO: 23, SEQ ID NO: 24, SEQ ID NO: 25 or SEQ ID NO: 26.

13. An expression vector comprising a polynucleotide according to claim 11 or claim 12.

14. A host cell comprising an expression vector according to claim 13.

15. A method for expressing an α-amylase comprising:
providing a host cell comprising an expression vector according to claim 13, wherein the polynucleotide encoding the α-amylase is fused in-frame to a polynucleotide encoding a signal sequence;
expressing the α-amylase as a secreted polypeptide into host cell media; and
recovering the secreted α-amylase from the host cell growth media;
thereby isolating the α-amylase as a secreted polypeptide.

16. The method of claim 15, wherein the signal sequence is the native signal sequence, or is from *Bacillus* AmyE or AprE or *Streptomyces* CelA.

## Patentansprüche

1. Isolierte α-Amylase mit einer Aminosäure-Sequenzidentität von mindestens 80% zu AmyDSM90 (SEQ ID Nr.: 1), wobei die α-Amylase aufweist:
a) eine Asparaginsäure in einer Position, die Position 21 von AmyDSM90 (SEQ ID Nr.: 1) entspricht;
b) ein Asparagin in einer Position, die Position 97 von AmyDSM90 (SEQ ID Nr.: 1) entspricht, und
c) ein Isoleucin in einer Position, die Position 128 von AmyDSM90 (SEQ ID Nr.: 1) entspricht.

2. α-Amylase nach Anspruch 1, die eine Identität von mindestens 90% mit der Aminosäuresequenz von SEQ ID Nr.: 1 aufweist.

3. α-Amylase nach Anspruch 1, die eine Identität von mindestens 95% mit der Aminosäuresequenz von SEQ ID Nr.: 1 aufweist.

4. α-Amylase nach Anspruch 1, die eine Deletion der Reste aufweist, die R179 und G180 von AmyDSM90 (SEQ ID Nr.: 1) entsprechen.

5. α-Amylase nach Anspruch 1 oder Anspruch 4, die eine andere Aminosäure als Methionin in einer Position aufweist, die Position 200 von AmyDSM90 (SEQ ID Nr.: 1) entspricht.

6. α-Amylase nach Anspruch 1, welche die Aminosäuresequenz von SEQ ID Nr.: 1, SEQ ID Nr.: 28, SEQ ID Nr.: 29 oder SEQ ID Nr.: 30 aufweist.

7. Zusammensetzung, die das Polypeptid nach einem der Ansprüche 1-6 aufweist.

8. Zusammensetzung nach Anspruch 7, wobei die Zusammensetzung eine Reinigungs-zusammensetzung ist, z. B. wobei die Zusammensetzung die Entfernung von Stärkeflecken aus Wäsche, von Geschirr oder einer Textilie bewirkt.

9. Verfahren zum Entfernen eines Stärkeflecks von einer Oberfläche, wobei das Verfahren aufweist:
Inkubieren der Oberfläche in Anwesenheit einer wässrigen Zusammensetzung, die eine wirksame Menge einer α-Amylase nach einem der Ansprüche 1 bis 6 aufweist, und Zulassen, dass die α-Amylase in dem Stärkefleck vorhandene Stärkekomponenten hydrolysiert, um kleinere Monomere auf Stärkebasis zu erzeugen, die sich in der wässrigen Zusammensetzung auflösen, wodurch der Stärkefleck von der Oberfläche entfernt wird.

10. Verfahren nach Anspruch 9, wobei die Oberfläche eine textile Oberfläche, eine Geschirroberfläche oder eine Wäscheoberfläche ist.

11. Isoliertes Polynucleotid, das für eine α-Amylase nach einem der Ansprüche 1 bis 6 codiert.

12. Isoliertes Polynucleotid nach Anspruch 11, das die Sequenz von SEQ ID Nr.: 23, SEQ ID Nr.: 24, SEQ ID Nr.: 25 oder SEQ ID Nr.: 26 aufweist.

13. Expressionsvektor, der ein Polynucleotid nach Anspruch 11 oder Anspruch 12 aufweist.

14. Wirtszelle, die einen Expressionsvektor nach Anspruch 13 aufweist.

15. Verfahren zum Exprimieren einer α-Amylase, wobei das Verfahren aufweist:
Bereitstellen einer Wirtszelle, die einen Expressionsvektor nach Anspruch 13 aufweist, wobei das für die α-Amylase codierende Polynucleotid "in frame" an ein für eine Signalsequenz codierendes Polynucleotid fusioniert ist;
Exprimieren der α-Amylase als sezerniertes Polypeptid in das Wirtszellen-Medium; und
Rückgewinnen der sezernierten α-Amylase aus dem Wirtszellen-Wachstumsmedium;
dadurch Isolieren der α-Amylase als sezerniertes Polypeptid.

16. Verfahren nach Anspruch 15, wobei die Signalsequenz die native Signalsequenz ist oder von Bacillus AmyE oder AprE oder Streptomyces CelA herrührt.

## Revendications

1. α-amylase isolée possédant au moins 80% d'identité de séquence d'acides aminés avec AmyDSM90 (SEQ ID NO:1) et possédant:
a) un acide aspartique à une position correspondant à la position 21 de AmyDSM90 (SEQ ID NO:1);
b) une asparagine à une position correspondant à la position 97 de AmyDSM90 (SEQ ID NO:1); et
c) une isoleucine à une position correspondant à la position 128 de AmyDSM90 (SEQ ID NO:1).

2. α-amylase selon la revendication 1, possédant au moins 90% d'identité avec la séquence d'acides aminés de la SEQ ID NO:1.

3. α-amylase selon la revendication 1, possédant au moins 95% d'identité avec la séquence d'acides aminés de la SEQ ID NO:1.

4. α-amylase selon la revendication 1, possédant une délétion des résidus correspondant à R179 et G180 de AmyDSM90 (SEQ ID NO:1).

5. α-amylase selon la revendication 1 ou la revendication 4, possédant un acide aminé autre qu'une méthionine à une position correspondant à la position 200 de AmyDSM90 (SEQ ID NO:1).

6. α-amylase selon la revendication 1, possédant la séquence d'acides aminés de la SEQ ID NO:1, la SEQ ID NO:28, la SEQ ID NO:29 ou la SEQ ID NO:30.

7. Composition comprenant le polypeptide selon l'une quelconque des revendications 1-6.

8. Composition selon la revendication 7, où la composition est une composition nettoyante, par ex. où la composition est efficace pour retirer des taches d'amidon de blanchisserie, de vaisselle ou d'un textile.

9. Procédé pour le retrait d'une tache d'amidon à partir d'une surface, consistant:
à incuber la surface en présence d'une composition aqueuse comprenant une quantité efficace d'une α-amylase selon l'une quelconque des revendications 1 à 6 et à permettre à l'α-amylase d'hydrolyser les composants d'amidon présents dans la tache d'amidon pour produire des molécules dérivées d'amidon plus petites qui se dissolvent dans la composition aqueuse, retirant ainsi la tache d'amidon de la surface.

10. Procédé selon la revendication 9, dans lequel la surface est une surface de textile, une surface de vaisselle ou une surface de blanchisserie.

11. Polynucléotide isolé codant pour une α-amylase selon l'une quelconque des revendications 1 à 6.

12. Polynucléotide isolé selon la revendication 11, possédant la séquence de la SEQ ID NO:23, la SEQ ID NO:24, la SEQ ID NO:25 ou la SEQ ID NO:26.

13. Vecteur d'expression comprenant un polynucléotide selon la revendication 11 ou la revendication 12.

14. Cellule hôte comprenant un vecteur d'expression selon la revendication 13.

15. Procédé pour l'expression d'une α-amylase comprenant:
la fourniture d'une cellule hôte comprenant un vecteur d'expression selon la revendication 13, dans lequel le polynucléotide codant pour l'α-amylase est fusionné en cadre à un polynucléotide codant pour une séquence signal;
l'expression de l'α-amylase sous forme d'un polypeptide sécrété dans le milieu de la cellule hôte; et
la récupération de l'α-amylase sécrétée à partir du milieu de croissance de la cellule hôte;
isolant ainsi l'α-amylase sous forme d'un polypeptide sécrété.

16. Procédé selon la revendication 15, dans lequel la séquence signal est la séquence signal naturelle ou est issue de AmyE ou AprE de *Bacillus* ou de CelA de *Streptomyces.*
